# FASCICULE DE BREVET EUROPEEN

(11) **EP 0 780 118 B1**
(45) Date de publication et mention de la délivrance du brevet: **29.12.1997**
(21) Numéro de dépôt: 96402297.4
(22) Date de dépôt: 29.10.1996
(51) Int. Cl.: A61K 7/13, C07D 209/08, C07D 209/12, C07D 209/30, C07D 209/42, C07D 409/04, C07D 405/04, C07F 9/572

(54) **Compositions de teinture des fibres kératiniques contenant des dérivés N-substitués de la 4-hydroxy indoline, et certains de ces dérivés**
Zusammensetzungen zum Färben von Keratinfasern, die N-substituierte 4-Hydroxyindolinderivate enthalten, und verschiedene dieser Derivate
Compositions for dyeing keratinous fibers containing N-substituted 4-hydroxy indoline derivatives, and certain of these derivatives

(30) Priorité: 06.12.1995 FR 9514372
(43) Date de publication de la demande: 25.06.1997
(73) Titulaire: L'OREAL, 75008 Paris (FR)
(72) Inventeur: Terranova, Eric, 92600 Asnieres (FR); Fadli, Aziz, 93150 Le Blanc-Mesnil (FR); Lagrange, Alain, 77450 Coupvray (FR)
(74) Mandataire: Miszputen, Laurent

(56) Documents cités:
- EP-A- 0 285 179
- EP-A- 0 604 278
- FR-A- 2 008 797

## Description

L'invention a pour principal objet une composition pour la teinture d'oxydation des fibres kératiniques et en particulier des fibres kératiniques humaines telles que les cheveux contenant, à titre de coupleur, au moins un dérivé N-substitué de la 4-hydroxy indoline, et au moins une base d'oxydation.

Il est connu de teindre les fibres kératiniques et en particulier les cheveux humains avec des compositions tinctoriales contenant des précurseurs de colorant d'oxydation, en particulier des ortho ou paraphénylènediamines, des ortho ou paraaminophénols, des composés hétérocycliques, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques tels que des composés indoliniques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

Il a déjà été proposé, notamment dans la demande de brevet français FR-A-2 008 797, des compositions pour la teinture d'oxydation des fibres kératiniques renfermant, à titre de coupleurs, au moins un dérivé d'indoline et notamment au moins un dérivé de la 4-hydroxy indoline pouvant être N-substitué par un radical alkyle en C₁-C₄. De telles compositions permettent d'atteindre des gammes variées de nuances mais elles ne sont cependant pas entièrement satisfaisantes notamment du point de vue de la tenue des colorations obtenues vis à vis des diverses agressions que peuvent subir les cheveux et en particulier vis à vis de la lumière et des shampooings.

Or, la demanderesse vient maintenant de découvrir qu'il est possible d'obtenir de nouvelles teintures puissantes, peu sélectives et particulièrement résistantes, capables d'engendrer des colorations intenses dans des nuances variées, en utilisant des dérivés particuliers de la 4-hydroxy indoline, à savoir des dérivés convenablement N-substitués. Ces composés (pour partie nouveaux en soi) sont de plus aisément synthétisables.

Cette découverte est à la base de la présente invention.

L'invention a donc pour objet une composition pour la teinture des fibres kératiniques et en particulier des fibres kératiniques humaines telles que les cheveux, caractérisée par le fait qu'elle comprend, dans un milieu approprié pour la teinture:
- à titre de coupleur, au moins un dérivé N-substitué de la 4-hydroxy indoline de formule (I), et/ou au moins l'un de ses sels d'addition avec un acide : 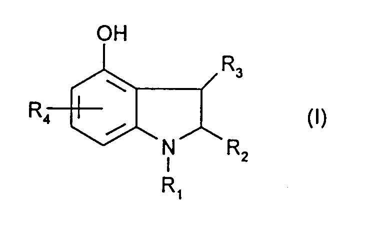 dans laquelle :
   - R₁ représente un radical monohydroxyalkyle en C₁-C₄ ; polyhydroxyalkyle en C₂-C₄ ; alcoxy(C₁-C₄)alkyle en C₁-C₄ ; hydroxyalcoxy(C₁-C₄)alkyle en C₁-C₄ : acétyle, aminoalkyle en C₁-C₄ ; aminoalkyle en C₁-C₄ dont l'amine est mono ou disubstituée par un groupement alkyle en C₁-C₄, par un groupement acétyle, par un groupement monohydroxyalkyle en C₁-C₄ ou par un groupement polyhydroxyalkyle en C₂-C₄ ; alkyle(C₁-C₄)thioalkyle en C₁-C₄, monohydroxyalkyl(C₁-C₄)thioalkyle en C₁-C₄ ; polyhydroxyalkyl(C₂-C₄)thioalkyle en C₁-C₄ ; carboxyalkyle en C₁-C₄ ; alcoxy(C₁-C₄)carbonylalkyle en C₁-C₄, acétylaminoalkyle en C₁-C₄ ; cyanoalkyle en C₁-C₄ ; trifluoroalkyle en C₁-C₄ ; halogénoalkyle en C₁-C₄ ; phosphoalkyle en C₁-C₄ ou sulfoalkyle en C₁-C₄ ;
   - R₂ et R₃, identiques ou différents, représentent un atome d'hydrogène ou d'halogène ; un radical alkyle en C₁-C₄ ; carboxyle ; alcoxy(C₁-C₄)carbonyle ou formyle ;
   - R₄ représente un atome d'hydrogène ou d'halogène ; un radical alkyle en C₁-C₄ ; alcoxy en C₁-C₄ ; acétylamino ; monohydroxyalkyle en C₁-C₅ ; polyhydroxyalkyle en C₂-C₄ ; alcoxy(C₁-C₄)alkyle en C₁-C₄ ; thiophène ; furane ; phényle ; aralkyle en C₁-C₄ ; phényle ou aralkyle en C₁-C₄ substitué par un atome d'halogène, un alkyle en C₁-C₄, un trifluorométhyle, un alcoxy en C₁-C₄, un amino ou par un amino mono- ou disubstitué par un radical alkyle en C₁-C₄ ; alkyl(C₁-C₄)aminoalkyle en C₁-C₄ ou dialkyl(C₁-C₄)aminoalkyle en C₁-C₄ ;
- et au moins une base d'oxydation.

Dans la formule (I) ci-dessus, les groupements alkyle en C₁-C₄ et alcoxy en C₁-C₄ peuvent être linéaires ou ramifiés et parmi les atomes d'halogène, on peut citer le chlore, le brome, l'iode et le fluor.

Les coupleurs de formule (I) conforme à l'invention se démarquent des produits connus du document FR-A-2 008 797 précité, essentiellement par la nature du substituant R₁ en position 1-N.

De préférence, selon la présente invention, le substituant R₁ est choisi parmi les radicaux monohydroxyalkyle en C₁-C₄ et les radicaux polyhydroxyalkyle en C₂-C₄.

Les colorations obtenues avec la composition de teinture conforme à l'invention, sont de nuances variées, puissantes, peu sélectives et présentent d'excellentes propriétés de résistance à la fois vis à vis des agents atmosphériques tels que la lumière et les intempéries et vis à vis de la transpiration et des différents traitements que peuvent subir les cheveux (shampooings, déformations permanentes). Ces propriétés sont particulièrement remarquables notamment vis à vis de la lumière et des shampooings.

Parmi les dérivés N-substitués de la 4-hydroxy indoline de formule (I), utilisables à titre de coupleurs dans les compositions conformes à l'invention, on peut notamment citer : >
- la 4-hydroxy 1-N-(β-hydroxyéthyl) indoline,
- la 4-hydroxy 1-N-(β-hydroxypropyl) indoline,
- la 1-N-acétyl 4-hydroxy indoline,
- la 1-N-(β,γ-dihydroxypropyl) 4-hydroxy indoline,
- la 4-hydroxy 1-N-(β-hydroxyéthyl) 5-méthyl indoline,
- la 4-hydroxy 1-N-(β-hydroxypropyl) 5-méthyl indoline,
- la 1-N-(β,γ-dihydroxypropyl) 4-hydroxy 5-méthyl indoline,
- la 4-hydroxy 1-N-(β-hydroxyéthyl) 6-méthyl indoline,
- la 4-hydroxy 1-N-(β-hydroxypropyl) 6-méthyl indoline,
- la 1-N-(β,γ-dihydroxypropyl) 4-hydroxy 6-méthyl indoline,
- la 5-benzyl 4-hydroxy 1-N-(β-hydroxyéthyl) indoline,
- la 5-benzyl 4-hydroxy 1-N-(β-hydroxypropyl) indoline,
- la 5-benzyl 1-N-(β,γ-dihydroxypropyl) 4-hydroxy indoline,
- la 4-hydroxy 1-N-(β-hydroxyéthyl) 5-β-hydroxyéthyl indoline,
- la 4-hydroxy 5-β-hydroxyéthyl 1-N-(β-hydroxypropyl) indoline,
- la 1-N-(β,γ-dihydroxypropyl) 4-hydroxy 5-β-hydroxyéthyl indoline,
- la 4-hydroxy 1-N-(β-hydroxyéthyl) 5-β,γ-dihydroxypropyl indoline,
- la 4-hydroxy 1-N-(β-hydroxypropyl) 5-β,γ-dihydroxypropyl indoline,
- la 1-N-(β,γ-dihydroxypropyl) 4-hydroxy 5-β,γ-dihydroxypropyl indoline,
- la 1-N-(γ-diméthylaminopropyl) 4-hydroxy indoline,
- la 1-N-éthylaminoéthyl 4-hydroxy indoline
et leurs sels d'addition avec un acide.

Parmi ces dérivés N-substitués de la 4-hydroxy indoline, on préfère plus particulièrement :
- la 4-hydroxy 1-N-(β-hydroxyéthyl) indoline,
- la 4-hydroxy 1-N-(β-hydroxypropyl) indoline,
- la 1-N-acétyl 4-hydroxy indoline,
- la 1-N-(β,γ-dihydroxypropyl) 4-hydroxy indoline,
- la 4-hydroxy 1-N-(β-hydroxyéthyl) 5-méthyl indoline,
- la 1-N-(γ-diméthylaminopropyl) 4-hydroxy indoline,
et leur sels d'addition avec un acide.

Les sels d'addition avec un acide des composés de formule (I) utilisables à titre de coupleurs dans les compositions de teinture conformes à l'invention sont notamment choisis parmi les chlorhydrates, les bromhydrates, les sulfates et les tartrates.

Le ou les dérivés N-substitués de la 4-hydroxy indoline de formule (I) représentent de préférence de 0,0005 à 12 % en poids environ du poids total de la composition tinctoriale, et encore plus préférentiellement de 0,005 à 6 % en poids environ de ce poids.

La nature de la ou des bases d'oxydation pouvant être utilisées dans la composition tinctoriale selon l'invention n'est pas critique. Cette ou ces bases d'oxydation sont de préférence choisies parmi les paraphénylènediamines, les bis-phénylalkylènediamines, les para-aminophénols, les ortho-aminophénols, les bases hétérocycliques, et leurs sels d'addition avec un acide.

Parmi les paraphénylènediamines utilisables à titre de bases d'oxydation dans la composition tinctoriale selon l'invention, on peut notamment citer les composés répondant à la formule (II) suivante, et leurs sels d'addition avec un acide : 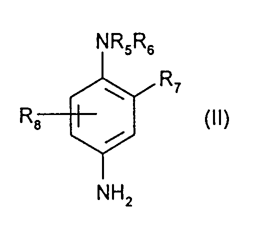 dans laquelle :
R₅ représente un atome d'hydrogène, un radical alkyle en C₁-C₄, monohydroxyalkyle en C₁-C₄, polyhydroxyalkyle en C₂-C₄ ou alcoxy(C₁-C₄)alkyle(C₁-C₄),
R₆ représente un atome d'hydrogène, un radical alkyle en C₁-C₄, monohydroxyalkyle en C₁-C₄ ou polyhydroxyalkyle en C₂-C₄,
R₇ représente un atome d'hydrogène, un atome d'halogène tel qu'un atome de chlore, un radical alkyle en C₁-C₄, sulfo, carboxy, monohydroxyalkyle en C₁-C₄ ou hydroxyalcoxy en C₁-C₄,
R₈ représente un atome d'hydrogène ou un radical alkyle en C₁-C₄.

Dans la formule (II) ci-dessus, et lorsque R₇ est différent d'un atome d'hydrogène, alors R₅ et R₆ représentent de préférence un atome d'hydrogène et R₇ est de préférence identique à R₈, et lorsque R₇ représente un atome d'halogéne, alors R₅, R₆ et R₈ représentent de préférence un atome d'hydrogène.

Parmi les paraphénylènediamines de formule (II) ci-dessus, on peut plus particulièrement citer la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, le 4-amino 1-(β-méthoxyéthyl)amino benzène, la 2-chloro paraphénylènediamine, et leurs sels d'addition avec un acide.

Parmi les bis-phénylalkylènediamines utilisables à titre de bases d'oxydation dans la composition tinctoriale selon l'invention, on peut notamment citer les composés répondant à la formule (III) suivante, et leurs sels d'addition avec un acide : 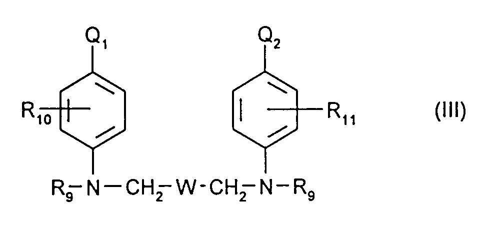 dans laquelle :
Q₁ et Q₂, identiques ou différents, représentent un radical hydroxyle ou NHR₁₂ dans lequel R₁₂ représente un atome d'hydrogène ou un radical alkyle en C₁-C₄,
R₉ représente un atome d'hydrogène, un radical alkyle en C₁-C₄, monohydroxyalkyle en C₁-C₄, polyhydroxyalkyle en C₂-C₄ ou aminoalkyle en C₁-C₄ dont le reste amino peut être substitué,
R₁₀ et R₁₁, identiques ou différents, représentent un atome d'hydrogène ou d'halogène ou un radical alkyle en C₁-C₄,
W représente un radical pris dans le groupe constitué par les radicaux suivants : -(CH₂)ₙ-; -(CH₂)ₘ-O-(CH₂)ₘ-; -(CH₂)m-CHOH-(CH₂)ₘ- et 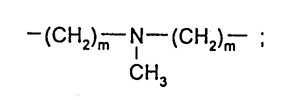 dans lesquels n est un nombre entier compris entre 0 et 8 inclusivement et m est un nombre entier compris entre 0 et 4 inclusivement.

Parmi les bis-phénylalkylènediamines de formule (III) ci-dessus, on peut plus particulièrement citer le N,N'-bis-(ß-hydroxyéthyl) N,N'-bis(4'-aminophényl) 1,3-diamino 2-propanol, la N,N'-bis-(ß-hydroxyéthyl) N,N'-bis-(4'-aminophényl) éthylènediamine, la N,N'-bis-(4-aminophényl) tétraméthylènediamine, la NN'-bis-(ß-hydroxyéthyl) N,N'-bis-(4-aminophényl) tétraméthylènediamine, la N,N'-bis-(4-méthylaminophényl) tétraméthylènediamine, la N,N'-bis-(éthyl) N,N'-bis-(4'-amino 3'-méthylphényl) éthylènediamine, et leurs sels d'addition avec un acide.

Parmi ces bis-phénylalkylènediamines de formule (III), le N,N'-bis-(ß-hydroxyéthyl) N,N'-bis-(4'-aminophényl) 1,3-diamino 2-propanol ou l'un de ses sels d'addition avec un acide sont particulièrement préférés.

Parmi les para-aminophénols utilisables à titre de bases d'oxydation dans la composition tinctoriale selon l'invention, on peut notamment citer les composés répondant à la formule (IV) suivante, et leurs sels d'addition avec un acide : 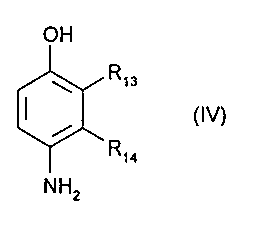 dans laquelle :
R₁₃ représente un atome d'hydrogène, un radical alkyle en C₁-C₄, monohydroxyalkyle en C₁-C₄, alcoxy(C₁-C₄)alkyle(C₁-C₄), hydroxyalkyl(C₁-C₄)aminoalkyle en C₁-C₄ ou aminoalkyle en C₁-C₄ ;
R₁₄ représente un atome d'hydrogène ou de fluor, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄);
étant entendu qu'au moins un des radicaux R₁₃ ou R₁₄ représente un atome d'hydrogène.

Parmi les para-aminophénols de formule (IV) ci-dessus, on peut plus particulièrement citer le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthylaminométhyl) phénol, et leurs sels d'addition avec un acide.

Parmi les ortho-aminophénols utilisables à titre de bases d'oxydation dans la composition tinctoriale selon l'invention, on peut notamment citer le 2-amino phénol, le 2-amino 1-hydroxy 5-méthyl benzène, le 2-amino 1-hydroxy 6-méthyl benzène, le 5-acétamido 2-amino phénol, et leurs sels d'addition avec un acide.

Parmi les bases hétérocycliques utilisables à titre de bases d'oxydation dans la composition tinctoriale selon l'invention, on peut notamment citer les dérivés pyridiniques, les dérivés pyrimidiniques, les dérivés pyrazoliques, et leurs sels d'addition avec un acide.

Parmi les dérivés pyridiniques, on peut plus particulièrement citer les composés décrits par exemple dans les brevets GB 1 026 978 et GB 1 153 196, comme la 2,5-diaminopyridine, et leurs sels d'addition avec un acide.
Parmi les dérivés pyrimidiniques, on peut plus particulièrement citer les composés décrits par exemple dans les brevets allemand DE 2 359 399 ou japonais JP 88-169 571 et JP 91-333 495, comme la 2,4,5,6-tétra-aminopyrimidine, la 4-hydroxy 2,5,6-triaminopyrimidine, et leurs sels d'addition avec un acide.
Parmi les dérivés pyrazoliques, on peut plus particulièrement citer les composés décrits dans les brevets DE 3 843 892, DE 4 133 957 et demandes de brevet WO 94/08969 et WO 94/08970 comme le 4,5-diamino 1-méthyl pyrazole, le 3,4-diamino pyrazole, et leurs sels d'addition avec un acide.

Selon l'invention, la ou les bases d'oxydation représentent de préférence de 0,0005 à 12 % en poids environ du poids total de la composition tinctoriale, et encore plus préférentiellement de 0,005 à 6 % en poids environ de ce poids.

La composition tinctoriale selon l'invention peut également renfermer un ou plusieurs coupleurs additionnels différents des dérivés N-substitués de la 4-hydroxy indoline de formule (I) et/ou un ou plusieurs colorants directs de façon à faire varier ou enrichir en reflets les nuances obtenues avec les bases d'oxydation.

Les coupleurs additionnels utilisables dans la composition selon l'invention peuvent être choisis parmi les coupleurs utilisés de façon classique en teinture d'oxydation et parmi lesquels on peut notamment citer les métaphénylènediamines, les méta-aminophénols, les métadiphénols et les coupleurs hétérocycliques tels que par exemple les dérivés indoliques, les dérivés indoliniques, et leurs sels d'addition avec un acide.

Ces coupleurs peuvent notamment être choisis parmi le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxy benzène, le 1,3-diamino benzène, le 1,3-bis-(2,4-diaminophénoxy) propane, le sésamol, l'α-naphtol, le 6-hydroxy indole, le 4-hydroxy indole, le 4-hydroxy N-méthyl indole, la 6-hydroxy indoline, et leurs sels d'addition avec un acide.

Lorsqu'ils sont présents ces coupleurs additionnels représentent de préférence de 0,0005 à 5 % en poids environ du poids total de la composition tinctoriale, et encore plus préférentiellement de 0,005 à 3 % en poids environ de ce poids.

Les sels d'addition avec un acide de la ou des bases d'oxydation et/ou des coupleurs additionnels utilisables dans la composition tinctoriale de l'invention sont notamment choisis parmi les chlorhydrates, les bromhydrates, les sulfates et les tartrates, les lactates et les acétates.

Le milieu approprié pour la teinture (ou support) est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; le glycérol; les glycols et éthers de glycols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 3 et 12. Il peut être ajusté à la valeur désirée au moyen d'agents acidifiants ou alcalinisants habituellement utilisés en teinture des fibres kératiniques.

Parmi les agents acidifiants, on peut citer, à titre d'exemple, les acides minéraux ou organiques comme l'acide chlorhydrique, l'acide orthophosphorique, les acides carboxyliques comme l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (V) suivante : 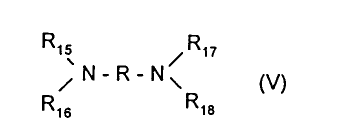 dans laquelle R est un reste propylène éventuellement substitué par un groupement hydroxyle ou un radical alkyle en C₁-C₄ ; R₁₅, R₁₆, R₁₇ et R₁₈, identiques ou différents, représentent un atome d'hydrogène, un radical alkyle en C₁-C₄ ou hydroxyalkyle en C₁-C₄.

La composition tinctoriale selon l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents de conditionnement tels que par exemple des silicones, des agents filmogènes, des agents conservateurs, des agents opacifiants.

Bien entendu, l'homme de l'art veillera à choisir le ou les éventuels composés complémentaires mentionnés ci-avant, de manière telle que les propriétés avantageuses attachées intrinsèquement à la composition tinctoriale selon l'invention ne soient pas, ou substantiellement pas, altérées par la ou les adjonctions envisagées.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

L'invention a également pour objet l'utilisation des dérivés N-substitués de la 4-hydroxy indoline de formule (I) ci-dessus, à titre de coupleur, en association avec au moins une base d'oxydation pour la teinture d'oxydation des fibres kératiniques et particulier des fibres kératiniques humaines telles que les cheveux.

Un autre objet de l'invention est un procédé de teinture d'oxydation des fibres kératiniques et en particulier des fibres kératiniques humaines telles que les cheveux mettant en oeuvre la composition tinctoriale telle que définie précédemment.

Selon ce procédé, on applique sur les fibres au moins une composition tinctoriale telle que définie précédemment, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

Selon une forme de mise en oeuvre particulièrement préférée du procédé de teinture selon l'invention, on mélange, au moment de l'emploi, la composition tinctoriale décrite ci-dessus avec une composition oxydante contenant, dans un milieu approprié pour la teinture, au moins un agent oxydant présent en une quantité suffisante pour développer une coloration. Le mélange obtenu est ensuite appliqué sur les fibres kératiniques et on laisse poser pendant 3 à 50 minutes environ, de préférence 5 à 30 minutes environ, après quoi on rince, on lave au shampooing, on rince à nouveau et on sèche.

L'agent oxydant présent dans la composition oxydante telle que définie ci-dessus peut être choisi parmi les agents oxydants classiquement utilisés pour la teinture d'oxydation des fibres kératiniques, et parmi lesquels on peut citer le peroxyde d'hydrogène, le peroxyde d'urée, les bromates de métaux alcalins, les persels tels que les perborates et persulfates. Le peroxyde d'hydrogène est particulièrement préféré.

Le pH de la composition oxydante renfermant l'agent oxydant tel que défini ci-dessus est tel qu'après mélange avec la composition tinctoriale, le pH de la composition résultante appliquée sur les fibres kératiniques varie de préférence entre 3 et 12 environ et encore plus préférentiellement entre 5 et 11. Il est ajusté à la valeur désirée au moyen d'agents acidifiants ou alcalinisants habituellement utilisés en teinture des fibres kératiniques et tels que définis précédemment.

La composition oxydante telle que définie ci-dessus peut également renfermer divers adjuvants utilisés classiquement dans les compositions pour la teinture des cheveux et tels que définis précédemment.

La composition qui est finalement appliquée sur les fibres kératiniques peut se présenter sous des formes diverses, telles que sous forme de liquides, de crèmes, de gels, ou sous toute autre forme appropriée pour réaliser une teinture des fibres kératiniques, et notamment des cheveux humains.

Un autre objet de l'invention est un dispositif à plusieurs compartiments ou "kit" de teinture ou tout autre système de conditionnement à plusieurs compartiments dont un premier compartiment renferme la composition tinctoriale telle que définie ci-dessus et un second compartiment renferme la composition oxydante telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Certains composés de formule (I), utilisés à titre de coupleurs dans le cadre de la présente invention, sont nouveaux et, à ce titre, constituent un autre objet de l'invention.

Ces nouveaux dérivés N-substitués de la 4-hydroxy indoline et leurs sels d'addition avec un acide répondent à la formule (I') suivante : 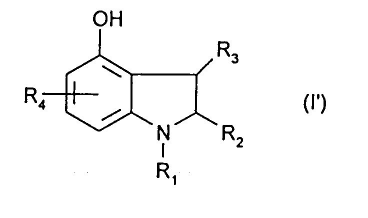 dans laquelle :
- R₁ représente un radical monohydroxyalkyle en C₁-C₄ ; polyhydroxyalkyle en C₂-C₄ ; alcoxy(C₁-C₄)alkyle en C₁-C₄ ; hydroxyalcoxy(C₁-C₄)alkyle en C₁-C₄ ; acétyle, aminoalkyle en C₁-C₄ ; aminoalkyle en C₁-C₄ dont l'amine est mono ou disubstituée par un groupement alkyle en C₁-C₄, par un groupement acétyle, par un groupement monohydroxyalkyle en C₁-C₄ ou par un groupement polyhydroxyalkyle en C₂-C₄ ; alkyle(C₁-C₄)thioalkyle en C₁-C₄, monohydroxyalkyl(C₁-C₄)thioalkyle en C₁-C₄ ; polyhydroxyalkyl(C₂-C₄)thioalkyle en C₁-C₄ ; carboxyalkyle en C₁-C₄ ; alcoxy(C₁-C₄)carbonylalkyle en C₁-C₄, acétylaminoalkyle en C₁-C₄ ; cyanoalkyle en C₁-C₄ ; trifluoroalkyle en C₁-C₄ ; halogénoalkyle en C₁-C₄ ; phosphoalkyle en C₁-C₄ ou sulfoalkyle en C₁-C₄ ;
- R₂ et R₃, identiques ou différents, représentent un atome d'hydrogène ou d'halogène ; un radical alkyle en C₁-C₄ ; carboxyle ; alcoxy(C₁-C₄)carbonyle ou formyle;
- R₄ représente un atome d'hydrogène ou d'halogéne ; un radical alkyle en C₁-C₄ ; alcoxy en C₁-C₄ ; acétylamino ; monohydroxyalkyle en C₁-C₅ ; polyhydroxyalkyle en C₂-C₄ ; alcoxy(C₁-C₄)alkyle en C₁-C₄ ; thiophène ; furane ; phényle ; aralkyle en C₁-C₄ ; phényle ou aralkyle en C₁-C₄ substitué par un atome d'halogène, un alkyle en C₁-C₄, un trifluorométhyle, un alcoxy en C₁-C₄, un amino ou par un amino mono- ou disubstitué par un radical alkyle en C₁-C₄ ; alkyl(C₁-C₄)aminoalkyle en C₁-C₄ ou dialkyl(C₁-C₄)aminoalkyle en C₁-C₄ ;
   étant entendu que :
- lorsque R₂, R₃ et R₄ représentent simultanément un atome d'hydrogène, alors R₁ ne peut représenter un radical méthoxycarbonylpropyle.

De préférence, selon la présente invention, le substituant R₁ est choisi parmi les radicaux monohydroxyalkyle en C₁-C₄ et les radicaux polyhydroxyalkyle en C₂-C₄.

Parmi les nouveaux dérivés N-substitués de la 4-hydroxy indoline de formule (I'), on peut notamment citer :
- la 4-hydroxy 1-N-(β-hydroxyéthyl) indoline,
- la 4-hydroxy 1-N-(β-hydroxypropyl) indoline,
- la 1-N-acétyl 4-hydroxy indoline,
- la 1-N-(β,γ-dihydroxypropyl) 4-hydroxy indoline,
- la 4-hydroxy 1-N-(β-hydroxyéthyl) 5-méthyl indoline,
- la 4-hydroxy 1-N-(β-hydroxypropyl) 5-méthyl indoline,
- la 1-N-(β,γ-dihydroxypropyl) 4-hydroxy 5-méthyl indoline,
- la 4-hydroxy 1-N-(β-hydroxyéthyl) 6-méthyl indoline,
- la 4-hydroxy 1-N-(β-hydroxypropyl) 6-méthyl indoline,
- la 1-N-(β,γ-dihydroxypropyl) 4-hydroxy 6-méthyl indoline,
- la 5-benzyl 4-hydroxy 1-N-(β-hydroxyéthyl) indoline,
- la 5-benzyl 4-hydroxy 1-N-(β-hydroxypropyl) indoline,
- la 5-benzyl 1-N-(β,γ-dihydroxypropyl) 4-hydroxy indoline,
- la 4-hydroxy 1-N-(β-hydroxyéthyl) 5-β-hydroxyéthyl indoline,
- la 4-hydroxy 5-β-hydroxyéthyl 1-N-(β-hydroxypropyl) indoline,
- la 1-N-(β,γ-dihydroxypropyl) 4-hydroxy 5-β-hydroxyéthyl indoline,
- la 4-hydroxy 1-N-(β-hydroxyéthyl) 5-β,γ-dihydroxypropyl indoline,
- la 4-hydroxy 1-N-(β-hydroxypropyl) 5-β,γ-dihydroxypropyl indoline,
- la 1-N-(β,γ-dihydroxypropyl) 4-hydroxy 5-β,γ-dihydroxypropyl indoline,
- la 1-N-(γ-diméthylaminopropyl) 4-hydroxy indoline,
- la 1-N-éthylaminoéthyl 4-hydroxy indoline
et leurs sels d'addition avec un acide.

Parmi ces nouveaux dérivés N-substitués du 4-hydroxy indoline de formule (I'), on préfère plus particulièrement :
- la 4-hydroxy 1-N-(β-hydroxyéthyl) indoline,
- la 4-hydroxy 1-N-(β-hydroxypropyl) indoline,
- la 1-N-acétyl 4-hydroxy indoline,
- la 1-N-(β,γ-dihydroxypropyl) 4-hydroxy indoline,
- la 4-hydroxy 1-N-(β-hydroxyéthyl) 5-méthyl indoline,
- la 1-N-(γ-diméthylaminopropyl) 4-hydroxy indoline,
et leurs sels d'addition avec un acide.

L'invention a également pour objet un procédé de préparation (procédé principal) des composés de formule (I'), répondant au schéma de synthèse suivant : 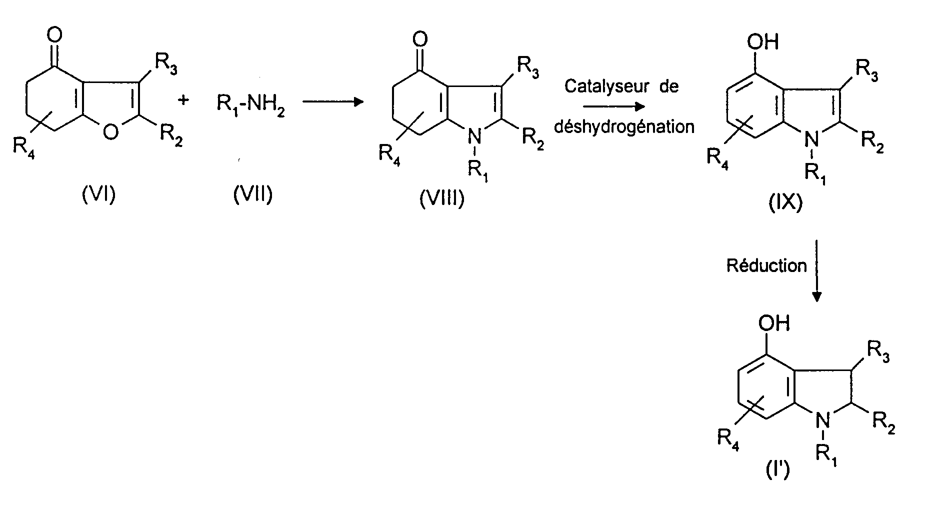 consistant à faire réagir, dans une première étape, un 4-oxo 4,5,6,7-tetrahydro benzofurane de formule (VI), dans laquelle les radicaux R₂, R₃ et R₄ ont la même signification que dans la formule (I') définie ci-dessus, avec une amine substituée de formule (VII), dans laquelle le radical R₁ a la même signification que dans la formule (I') définie ci-dessus, dans un milieu solvant dont la température est comprise de préférence entre 80 et 160°C, pour conduire à un dérivé 4-oxo 4,5,6,7-tetrahydroindolique de formule (VIII), dans laquelle les radicaux R₁, R₂, R₃ et R₄ ont la même signification que dans la formule (I') définie ci-dessus, puis dans une deuxième étape, à aromatiser le composé de formule (VIII) par déshydrogénation catalytique en milieu solvant, à une température généralement comprise entre 150 et 220°C et de préférence comprise entre 160 et 170°C, pour conduire à un composé de formule (IX) dans laquelle les radicaux R₁, R₂, R₃ et R₄ ont la même signification que dans la formule (I') définie ci-dessus, et enfin dans une troisième étape à réduire, en milieu acide et à une température généralement comprise entre 10 et 40°C, la double liaison du noyau pyrrolique du composé de formule (IX) à l'aide de réducteurs chimiques, pour conduire à un composé de formule (I') définie ci-dessus.

Parmi les solvants utilisés lors de la première étape, on peut plus particulièrement citer les alcools inférieurs tels que l'éthanol, le n-propanol, le 1-butanol, le 2-butanol, le 2-méthyl 1-butanol, le 3-méthyl 1-butanol, le 2-méthyl 1-propanol, le n-pentanol, le 2-pentanol, le 3-méthyl 3-pentanol, le 4-méthyl 2-pentanol ou encore le 2-éthyl 1-butanol.

Selon le procédé de l'invention, on peut employer tout catalyseur de déshydrogénation approprié, par exemple un métal choisi dans le groupe constitué par le manganèse, le platine, le palladium, le rhodium, le nickel et le ruthénium, leurs oxydes, et les combinaisons de ces substances.

Les catalyseurs de déshydrogénation préférés sont le palladium ou le platine. De façon connue, le catalyseur peut être déposé sur un support inerte. Parmi ces supports inertes, on peut citer par exemple le charbon de bois neutre, le charbon neutre, l'alumine neutre, les zéolithes, les argiles, etc. On utilise de préférence du charbon neutre.

Les catalyseurs de déshydrogénation sont en général présents en une quantité comprise entre 0,2 et 5 % en poids d'équivalent métal par rapport au poids du composé de formule (VIII) à faire réagir.

Les solvants utilisés lors de la deuxième étape sont de préférence choisis parmi les solvants dont le point d'ébullition est supérieur à 150°C, tel que par exemple le diglyme dont le point d'ébullition (P.E.) est d'environ 162°C et le diisobutylcétone (P.E. d'environ 169°C).

La réaction de réduction de la double liaison du noyau pyrrolique du composé de formule (IX), (troisième étape), est bien connue de l'homme du métier et est décrite par exemple dans la monographie de R.T. Brown, J.A. Joule et P.G. Sammes, "Comprehensive Organic Chemistry", vol. 4, p.443, Pergamon Press 1979.

Les solvants utilisés lors de cette troisième étape sont de préférence choisis parmi les acides carboxyliques tels que l'acide acétique, l'acide trifluoroacétique et l'acide trichloroacétique.

Selon une première variante de ce procédé principal, et lorsque dans la formule (VI) le radical R₄ est un atome d'hydrogène, alors on peut introduire un radical R'₄, en position 5, différent d'un atome d'hydrogène sur les composés de formule (VIII), par réaction d'aldolisation en milieu basique. Cette première variante répond au schéma de synthèse suivant : 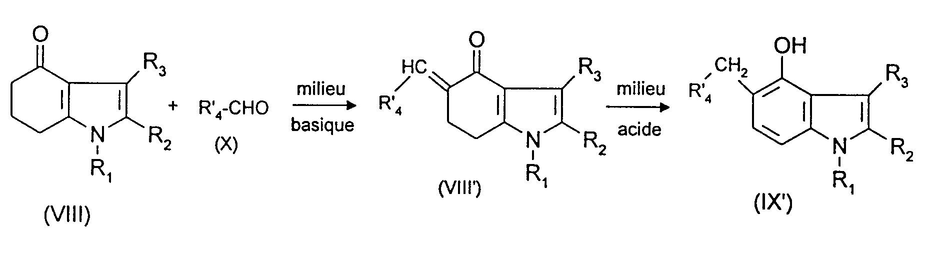 consistant à faire réagir un composé de formule (VIII) et un aldéhyde de formule (X) dans laquelle le radical R'₄ représente un radical alkyle en C₁-C₃, monohydroxyalkyle en C₁-C₄, polyhydroxyalkyle en C₂-C₃, alcoxyalkyle en C₁-C₃, alkyl(C₁-C₄)aminoalkyle en C₁-C₃ ou dialkyl(C₁-C₄)aminoalkyle en C₁-C₃, en milieu basique, pour obtenir un composé de formule (VIII') dans laquelle le radical R'₄ a la même signification que dans la formule (X), qui est ensuite isomérisé en milieu acide selon les conditions classiques d'isomérisation, pour conduire à un composé de formule (IX') dans laquelle les radicaux R₁, R₂ et R₃ ont les mêmes significations que dans la formule (I'), et le radical R'₄ a la même signification que dans la formule (X). Le composé de formule (IX') est ensuite réduit selon la méthode décrite pour le procédé principal, pour conduire à un composé de formule (I").

La réaction d'aldolisation est bien connue de l'homme du métier et est décrite par exemple dans la demande de brevet EP-A-0 377 450.

Selon une deuxième variante de ce procédé principal, on peut également fonctionnaliser la partie benzénique des composés de formule (IX) obtenus à partir d'un composé de formule (VIII) dans lequel R₄ représente un atome d'hydrogène pour introduire un radical R₄ différent d'un atome d'hydrogène. Cette seconde variante répond au schéma de synthèse suivant :

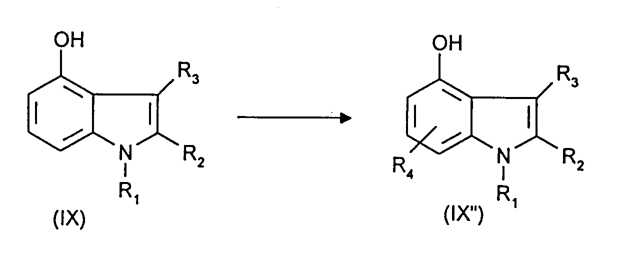

Le composé de formule (IX") est ensuite réduit selon la méthode décrite à la troisième étape du procédé principal, pour conduire à un composé de formule (I').

Cette seconde variante permet notamment d'introduire :
- soit un substituant R₄ de type dialkyl(C₁-C₄)aminométhyl, en position 5, par une réaction de MANNICH telle que décrite par exemple dans l'article de F. TROXLER & al., Helv. Chim. Acta, 51, (6), 1968 ;
- soit un substituant R₄ de type acétylamino par une réaction de nitration suivie d'une réaction de réduction et d'une acétylation, par les méthodes classiques bien connues de l'homme du métier;
- soit encore un substituant R₄ de type halogène, par une réaction classique d'halogénation, lorsque les radicaux R₂ et R₃ sont tous deux différents d'un atome d'hydrogène.

Cette énumération n'est bien sûr pas limitative du type de groupements R₄ pouvant être introduits directement sur les composés de formule (IX).

Les exemples qui suivent sont destinés à illustrer l'invention sans pour autant en limiter la portée.

### EXEMPLES DE PREPARATION

### EXEMPLE DE PREPARATION 1 : Synthèse de la 4-hydroxy 1-N-(β-hydroxypropyl) indoline

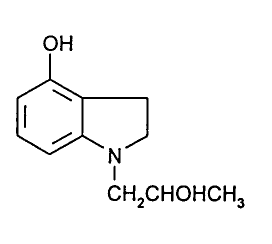

### a) Préparation du 4-oxo 4,5,6,7-tétrahydro 1-N-(β-hydroxypropyl) indole

A une solution de 13,6 g de 4-oxo 4,5,6,7-tetrahydro benzofurane dans 200 cm³ d'éthanol, on a ajouté 7,2 g de β-hydroxypropylamine. La solution a été portée à 150°C pendant 4 heures. L'éthanol a ensuite été évaporé sous vide. On a recueilli 18 g d'une huile qui a été utilisée telle quelle à l'étape suivante.

### b) Préparation du 4-hydroxy 1-N-(β-hydroxypropyl) indole

A une solution de 19,3 g de 4-Oxo 4,5,6,7-tetrahydro 1-N-(β-hydroxypropyl) indole, obtenu à l'étape précédente, dans 300 cm³ de diglyme, on a ajouté 10 g de palladium sur charbon à 5 % en poids et contenant 50 % d'eau. La température du milieu réactionnel a été portée à la température de reflux du diglyme pendant 5 heures, après élimination de l'eau par azéotropie.
Le catalyseur a ensuite été filtré sur celite puis on a évaporé le diglyme. On a obtenu 17 g de produit brut. Après chromatographie sur gel de silice (Heptane/acétate d'éthyle = 1/4), on a obtenu 15 g de produit visqueux qui a cristallisé par addition de 15 cm³ de dichlorométhane.
Après filtration, lavage à l'éther de pétrole et séchage sous vide et sur pentaoxyde de phosphore, on a récupéré 10 g de la 4-hydroxy 1-N-(β-hydroxypropyl) indole dont le point de fusion était compris entre 93 et 95°C.

### c) Préparation de la 4-hydroxy 1-N-(β-hydroxypropyl) indoline

A une solution de 7 g de 4-hydroxy 1-N-(β-hydroxypropyl) indole obtenu à l'étape précédente dans 40 cm³ d'acide acétique, on a ajouté 1,4 g de cyanoborohydrure de sodium, en maintenant la température en dessous de 30°C. Après 30 minutes d'agitation, le milieu réactionnel a été versé sur 200 g d'eau glacée et le pH a été neutralisé à 7,5 par ajout d'ammoniaque à 30 %. Le précipité obtenu a été filtré et séché. On a obtenu 4 g du produit attendu dont le point de fusion était compris entre 130 et 131°C et dont l'analyse élémentaire calculée pour C₁₁H₁₅NO₂ était la suivante :

| % | C | H | N | O |
|---|---|---|---|---|
| Calculée | 68,37 | 7,82 | 7,25 | 16,56 |
| Trouvée | 68,37 | 7,80 | 7,29 | 16,52 |

### EXEMPLE DE PREPARATION 2 : Synthèse de la 4-hydroxy 1-N-(β-hydroxyéthyl) indoline

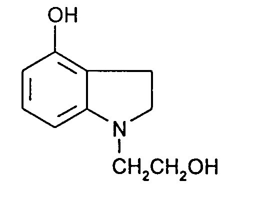

### a) Préparation du 4-oxo 4,5,6,7-tétrahydro 1-N-(β-hydroxyéthyl) indole

A une solution de 136 g de 4-oxo 4,5,6,7-tétrahydro benzofurane dans 250 cm³ d'éthanol, on a ajouté 80 g d'éthanolamine. La solution a été portée à 130°C pendant 6 heures. Après avoir laissé le mélange réactionnel revenir à température ambiante sous agitation, celui-ci a été coulé sur un mélange de 800 cm³ d'éther isopropylique et de 200 cm³ d'éther de pétrole. Un produit a cristallisé sous agitation, puis a été essoré, lavé à l'éther de pétrole et séché sous vide sur pentaoxyde de phosphore. On a recueilli 160 g du produit attendu qu'on a recristallisé dans 240 cm³ d'isopropanol. On a obtenu 150 g du produit attendu dont le point de fusion était compris entre 96 et 97°C.

### b) Préparation du 4-hydroxy 1-N-(β-hydroxyéthyl) indole

A une solution de 25 g de 4-oxo 4,5,6,7-tetrahydro 1-N-(β-hydroxyéthyl) indole, obtenu à l'étape précédente, dans 300 cm³ de diglyme, on a ajouté 15 g de palladium sur charbon à 5 % en poids et contenant 50 % d'eau. La température du mélange a été portée et maintenue à 162°C pendant 10 heures. On a ensuite laissé revenir le mélange à une température de 40°C, puis le catalyseur a été filtré. Les solvants ont ensuite été éliminés sous vide jusqu'à obtention de 21,4 g de produit brut qui ont été repris par un mélange de 30 cm³ de dichlorométhane et de 200 cm³ d'éther de pétrole. Les cristaux obtenus ont été essorés, lavés à l'éther de pétrole puis séchés sous vide sur pentaoxyde de phosphore. On a obtenu 11 g de la 4-hydroxy 1-N-(β-hydroxyéthyl) indole dont le point de fusion était de 109°C.

### c) Préparation de la 4-hydroxy 1-N-(β-hydroxyéthyl) indoline

A une solution de 8,85 g de 4-hydroxy 1-N-(β-hydroxyéthyl) indole obtenu à l'étape précédente dans 40 cm³ d'acide acétique, on a ajouté 1,9 g de cyanoborohydrure de sodium, en maintenant la température en dessous de 30°C. Après 30 minutes d'agitation, le milieu réactionnel a été versé sur 200 g d'eau glacée et le pH a été neutralisé à 7,5 par ajout d'ammoniaque à 30 %. Le précipité obtenu a été filtré et séché. On a obtenu 7,75 g de 4-hydroxy 1-N-(β-hydroxyéthyl) indoline dont le point de fusion était de 108°C. L'analyse en Résonnance Magnétique Nucléaire (R.M.N.) ¹³C et proton du produit obtenu a été trouvée conforme au produit attendu.

### EXEMPLE DE PREPARATION 3 : Synthèse du chlorhydrate de la 4-hydroxy 1-N-(β,γ-dihydroxypropyl) indoline

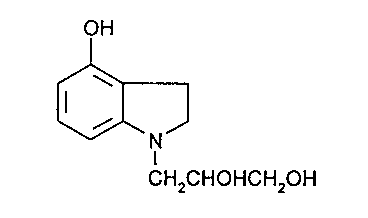

### a) Préparation du 4-oxo 4,5,6,7-tétrahydro 1-N-(β,γ-dihydroxypropyl) indole

A une solution de 13,6 g de 4-oxo 4,5,6,7-tetrahydro benzofurane dans 200 cm³ d'éthanol, on a ajouté 9,4 g de 2,3-dihydroxypropylamine. On a chauffé le milieu réactionnel à 150°C pendant 9 heures. Après évaporation du solvant et purification du brut sur gel de silice (Acétate d'éthyle/méthanol = 9/1), on a recueilli 11,2 g de 4-oxo 4,5,6,7-tetrahydro-1-N-(β,γ-dihydroxypropyl) indole sous forme d'une huile.

### b) Préparation du 4-hydroxy 1-N-(β,γ-dihydroxypropyl) indole

Dans une solution de 9,4 g de 4-oxo 4,5,6,7-tetrahydro 1-N-(β,γ-dihydroxypropyl) indole obtenu à l'étape précédente, dans 50 cm³ de diglyme, on a introduit 3,8 g de palladium sur charbon à 5% en poids et contenant 50 % d'eau. La température du milieu réactionnel a été portée à la température de reflux du diglyme, et l'eau a été distillée par azéotropie. Après 23 heures de réaction, le catalyseur a été filtré sur celite puis on a évaporé le diglyme sous vide. Le produit brut obtenu a été chromatographié sur gel de silice (Acétate d'éthyle/heptane = 9/1). On a obtenu 4,2 g de la 4-hydroxy 1-N-(β,γ-dihydroxypropyl) indole sous forme d'une huile très visqueuse.

### c) Préparation du chlorhydrate de 4-hydroxy 1-N-(β,γ-dihydroxypropyl) indoline

A une solution de 4 g de 4-hydroxy 1-N-(β,γ-dihydroxypropyl) indole obtenu à l'étape précédente dans 40 cm³ d'acide acétique, on a ajouté 0,7 g de cyanoborohydrure de sodium, en maintenant la température en dessous de 30°C. Après 30 minutes d'agitation, le milieu réactionnel a été versé sur 200 g d'eau glacée et le pH a été neutralisé à 7,5 par ajout d'ammoniaque à 30 %. Le précipité obtenu a été filtré et séché. Après traitement du précipité par une solution d'éthanol chlorhydrique 5,5 M, on a obtenu 3,4 g du produit attendu dont l'analyse élémentaire calculée pour C₁₁H₁₅NO₃, HCl était la suivante :

| % | C | H | N | O | Cl |
|---|---|---|---|---|---|
| Calculé | 53,77 | 6,56 | 5,7 | 19,53 | 14,43 |
| Trouvé | 52,91 | 6,53 | 5,68 | 18,96 | 14,82 |

### EXEMPLES DE FORMULATION

### EXEMPLES DE FORMULATION 1 (invention) et 2 (comparatif) :

On a préparé les compositions tinctoriales suivantes (teneurs en grammes) :

| **Compositions** | **1** | **2** **(*)** |
|---|---|---|
| 4-hydroxy 1-N-(β-hydroxyéthyl) indoline | 0,895 | |
| 4-hydroxy 1-N-méthyl indoline | | 0,745 |
| Paraphénylènediamine | 0,540 | 0,540 |
| Support de teinture commun | (**) | (**) |
| Eau déminéralisée q.s.p. | 100 g | 100 g |

| | | |
|---|---|---|
| (*) : exemple ne faisant pas partie de l'invention | | |
| (**) : support de teinture commun : | | |

| | |
|---|---|
| - Alcool oléique polyglycérolé à 2 moles de glycérol | 4,0 g |
| - Alcool oléique polyglycérolé à 4 moles de glycérol à 78 % de matières actives (M.A.) | 5,69 g M.A. |
| - Acide oléique | 3,0 g |
| - Amine oléique à 2 moles d'oxyde d'éthylène vendue sous la dénomination commerciale ETHOMEEN O12 par la société AKZO | 7,0 g |
| - Laurylamino succinamate de diéthylaminopropyle, sel de sodium à 55 % de M.A. | 3,0 g M.A. |
| - Alcool oléique | 5,0 g |
| - Diéthanolamide d'acide oléique | 12,0 g |
| - Propylèneglycol | 3,5 g |
| - Alcool éthylique | 7,0 g |
| - Dipropylèneglycol | 0,5 g |
| - Monométhyléther de propylèneglycol | 9,0 g |
| - Métabisulfite de sodium en solution aqueuse à 35 % de M.A. | 0,455 g M.A. |
| - Acétate d'ammonium | 0,8 g |
| - Antioxydant, séquestrant | q.s. |
| - Parfum, conservateur | q.s. |
| - Ammoniaque à 20 % de NH₃ | 10,0 g |

Au moment de l'emploi, chaque composition tinctoriale 1 et 2 a été mélangée avec un poids égal d'eau oxygénée à 20 volumes (6% en poids).

Chaque mélange obtenu a été appliqué pendant 30 minutes, sur des mèches de cheveux gris permanentés à 90 % de blancs. Après rinçage, lavage avec un shampooing standard et séchage, les mèches ont été teintes dans les nuances figurant dans le tableau I ci-dessous :

| **Exemple** | **1** | **2** **(*)** |
|---|---|---|
| **Nuance obtenue** | Châtain acajou irisé | Châtain acajou cuivré |

| | | |
|---|---|---|
| (*) : exemple ne faisant pas partie de l'invention | | |

Les mèches ainsi teintes ont ensuite subi un test de résistance à la lumière (Xenotest).

Pour ce faire, les mèches de cheveux teintes ont été fixées sur un support (carton ou plastique). Ces supports ont été disposés sur des porte-échantillons que l'on a fait tourner autour d'une lampe Xénon pendant une durée de 40 heures sous un taux d'humidité relative de 25 ± 5 % et à une température de 42,5 ± 2,5°C.

La couleur des mèches a été évaluée dans le système MUNSELL, avant et après le test de résistance à la lumière, au moyen d'un colorimètre CM 2002 MINOLTA.

Selon la notation MUNSELL, une couleur est définie par l'expression **H V / C** dans laquelle les trois paramètres désignent respectivement la teinte ou Hue (**H**), l'intensité ou Value (**V**) et la pureté ou Chromaticité (**C**), la barre oblique de cette expression est simplement une convention et n'indique pas un ratio.

La différence de couleur de chaque mèche avant et après le test de résistance à la lumière reflète la dégradation de la coloration due à l'action de la lumière et a été calculée en appliquant la formule de NICKERSON : **ΔE = 0,4 CoΔH + 6ΔV + 3 ΔC**, telle que décrite par exemple dans "Couleur, Industrie et Technique" : pages 14-17; vol. n° 5 ; 1978.

Dans cette formule, Δ**E** représente la différence de couleur entre deux mèches Δ**H**, Δ**V** et Δ**C** représentent la variation en valeur absolue des paramètres **H**, **V** et **C**, et **Co** représente la pureté de la mèche par rapport à laquelle on désire évaluer la différence de couleur (pureté de la mèche avant le test).

Les résultats sont donnés dans le tableau II ci-dessous :

| **EXEMPLE** | **Couleur avant le test** | **Couleur après le test** | **Dégradation de la coloration** | | | |
|---|---|---|---|---|---|---|
| | | | ΔH | ΔV | ΔC | ΔE |
| | | | | | | |
| **1** | 3,1 YR 2,4 / 1,7 | 4,0 YR 2,6 / 1,8 | 0,9 | 0,2 | 0,1 | **2,1** |
| **2****(*)** | 4,2 YR 2,5 / 1,9 | 5,5 YR 3,1 / 2,3 | 1,3 | 0,6 | 0,4 | **5,8** |

| | | | | | | |
|---|---|---|---|---|---|---|
| (*) : exemple ne faisant pas partie de l'invention | | | | | | |

On constate que la coloration obtenue avec la composition tinctoriale de l'exemple 1 selon l'invention (renfermant de la 4-hydroxy 1-N-(β-hydroxyéthyl) indoline) résiste beaucoup mieux à l'action de la lumière que la coloration obtenue avec la composition tinctoriale de l'exemple 2 ne faisant pas partie de l'invention car contenant la 4-hydroxy 1-N-méthyl indoline, composé ne répondant pas à la formule (I) définie précédemment, et correspondant à un composé de l'art antérieur tel que décrit dans la demande de brevet FR-A-2 008 797.

## Revendications

1. Composition pour la teinture des fibres kératiniques et en particulier des fibres kératiniques humaines telles que les cheveux, caractérisée par le fait qu'elle comprend, dans un milieu approprié pour la teinture :
- à titre de coupleur, au moins un dérivé N-substitué de la 4-hydroxy indoline de formule (I), et/ou au moins l'un de ses sels d'addition avec un acide : 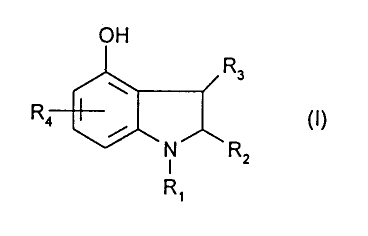 dans laquelle :
- R₁ représente un radical monohydroxyalkyle en C₁-C₄ ; polyhydroxyalkyle en C₂-C₄ ; alcoxy(C₁-C₄)alkyle en C₁-C₄ ; hydroxyalcoxy(C₁-C₄)alkyle en C₁-C₄ ; acétyle, aminoalkyle en C₁-C₄ ; aminoalkyle en C₁-C₄ dont l'amine est mono ou disubstituée par un groupement alkyle en C₁-C₄, par un groupement acétyle, par un groupement monohydroxyalkyle en C₁-C₄ ou par un groupement polyhydroxyalkyle en C₂-C₄ ; alkyle(C₁-C₄)thioalkyle en C₁-C₄, monohydroxyalkyl(C₁-C₄)thioalkyle en C₁-C₄ ; polyhydroxyalkyl(C₂-C₄)thioalkyle en C₁-C₄ ; carboxyalkyle en C₁-C₄ ; alcoxy(C₁-C₄)carbonylalkyle en C₁-C₄, acétylaminoalkyle en C₁-C₄ ; cyanoalkyle en C₁-C₄ ; trifluoroalkyle en C₁-C₄ ; halogénoalkyle en C₁-C₄ ; phosphoalkyle en C₁-C₄ ou sulfoalkyle en C₁-C₄ ;
- R₂ et R₃, identiques ou différents, représentent un atome d'hydrogène ou d'halogène : un radical alkyle en C₁-C₄ ; carboxyle ; alcoxy(C₁-C₄)carbonyle ou formyle ;
- R₄ représente un atome d'hydrogène ou d'halogène ; un radical alkyle en C₁-C₄ ; alcoxy en C₁-C₄ ; acétylamino ; monohydroxyalkyle en C₁-C₅ ; polyhydroxyalkyle en C₂-C₄ ; alcoxy(C₁-C₄)alkyle en C₁-C₄ ; thiophène ; furane ; phényle ; aralkyle en C₁-C₄ ; phényle ou aralkyle en C₁-C₄ substitué par un atome d'halogène, un alkyle en C₁-C₄, un trifluorométhyle, un alcoxy en C₁-C₄, un amino ou par un amino mono- ou disubstitué par un radical alkyle en C₁-C₄ ; alkyl(C₁-C₄)aminoalkyle en C₁-C₄ ou dialkyl(C₁-C₄)aminoalkyle en C₁-C₄ ; les groupements alkyle en C₁-C₄, et alcoxy en C₁-C₄ étant linéaires ou ramifiés et les atomes d'halogène pouvant être choisis parmi le chlore, le brome, l'iode et le fluor,
- et au moins une base d'oxydation.

2. Composition selon la revendication 1, caractérisée par le fait que le substituant R₁ est choisi parmi les radicaux monohydroxyalkyle en C₁-C₄ et les radicaux polyhydroxyalkyle en C₂-C₄.

3. Composition selon la revendication 1, caractérisée par le fait que les composés de formule (I) sont choisis parmi :
- la 4-hydroxy 1-N-(β-hydroxyéthyl) indoline,
- la 4-hydroxy 1-N-(β-hydroxypropyl) indoline,
- la 1-N-acétyl 4-hydroxy indoline,
- la 1-N-(β,γ-dihydroxypropyl) 4-hydroxy indoline,
- la 4-hydroxy 1-N-(β-hydroxyéthyl) 5-méthyl indoline,
- la 4-hydroxy 1-N-(β-hydroxypropyl) 5-méthyl indoline,
- la 1-N-(β,γ-dihydroxypropyl) 4-hydroxy 5-méthyl indoline,
- la 4-hydroxy 1-N-(β-hydroxyéthyl) 6-méthyl indoline,
- la 4-hydroxy 1-N-(β-hydroxypropyl) 6-méthyl indoline,
- la 1-N-(β,γ-dihydroxypropyl) 4-hydroxy 6-méthyl indoline,
- la 5-benzyl 4-hydroxy 1-N-(β-hydroxyéthyl) indoline,
- la 5-benzyl 4-hydroxy 1-N-(β-hydroxypropyl) indoline,
- la 5-benzyl 1-N-(β,γ-dihydroxypropyl) 4-hydroxy indoline,
- la 4-hydroxy 1-N-(β-hydroxyéthyl) 5-β-hydroxyéthyl indoline,
- la 4-hydroxy 5-β-hydroxyéthyl 1-N-(β-hydroxypropyl) indoline,
- la 1-N-(β,γ-dihydroxypropyl) 4-hydroxy 5-β-hydroxyéthyl indoline,
- la 4-hydroxy 1-N-(β-hydroxyéthyl) 5-β,γ-dihydroxypropyl indoline,
- la 4-hydroxy 1-N-(β-hydroxypropyl) 5-β,γ-dihydroxypropyl indoline,
- la 1-N-(β,γ-dihydroxypropyl) 4-hydroxy 5-β,γ-dihydroxypropyl indoline,
- la 1-N-(γ-diméthylaminopropyl) 4-hydroxy indoline,
- la 1-N-éthylaminoéthyl 4-hydroxy indoline
et leurs sels d'addition avec un acide.

4. Composition selon la revendication 3, caractérisée par le fait que les composés de formule (I) sont choisis parmi :
- la 4-hydroxy 1-N-(β-hydroxyéthyl) indoline,
- la 4-hydroxy 1-N-(β-hydroxypropyl) indoline,
- la 1-N-acétyl 4-hydroxy indoline,
- la 1-N-(β,γ-dihydroxypropyl) 4-hydroxy indoline,
- la 4-hydroxy 1-N-(β-hydroxyéthyl) 5-méthyl indoline,
- la 1-N-(γ-diméthylaminopropyl) 4-hydroxy indoline,
et leur sels d'addition avec un acide.

5. Composition selon l'une quelconque des revendications précédentes, caractérisée par le fait que les sels d'addition avec un acide des composés de formule (I) sont choisis parmi les chlorhydrates, les bromhydrates, les sulfates et les tartrates.

6. Composition selon l'une quelconque des revendications précédentes, caractérisée par le fait que le ou les composés de formule (I) représentent de 0,0005 à 12 % en poids du poids total de la composition tinctoriale.

7. Composition selon la revendication 6, caractérisée par le fait que le ou composés de formule (I) représentent de 0,005 à 6 % en poids du poids total de la composition tinctoriale.

8. Composition selon l'une quelconque des revendications précédentes, caractérisée par le fait que la ou les bases d'oxydation sont choisies parmi les paraphénylènediamines, les bis-phénylalkylènediamines, les para-aminophénols, les ortho-aminophénols, les bases hétérocycliques, et leurs sels d'addition avec un acide.

9. Composition selon l'une quelconque des revendications précédentes, caractérisée par le fait que la ou les bases d'oxydation représentent de 0,0005 à 12 % en poids environ du poids total de la composition tinctoriale.

10. Composition selon la revendication 9, caractérisée par le fait que la ou les bases d'oxydation représentent de 0,005 à 6 % en poids environ du poids total de la composition tinctoriale.

11. Composition selon l'une quelconque des revendications précédentes, caractérisée par le fait qu'elle renferme en outre un ou plusieurs coupleurs additionnels différents des dérivés N-substitués de la 4-hydroxy indoline de formule (I) et/ou un ou plusieurs colorants directs.

12. Composition selon l'une quelconque des revendications précédentes, caractérisée par le fait que le milieu approprié pour la teinture (ou support) est constitué par de l'eau ou par un mélange d'eau et d'au moins un solvant organique choisi parmi les alcanols inférieurs en C₁-C₄, le glycérol, les glycols et éthers de glycols, les alcools aromatiques, les produits analogues et leurs mélanges.

13. Composition selon l'une quelconque des revendications précédentes, caractérisée par le fait qu'elle présente un pH compris entre 3 et 12.

14. Composition selon l'une quelconque des revendications précédentes, caractérisée par le fait qu'elle se présente sous forme de liquides, de crèmes, de gels, ou sous toute autre forme appropriée pour réaliser une teinture des fibres kératiniques, et notamment des cheveux humains.

15. Utilisation des dérivés N-substitués de la 4-hydroxy indoline de formule (I) telle que définie à l'une quelconque des revendications 1 à 4, à titre de coupleurs dans des compositions pour la teinture d'oxydation des fibres kératiniques et en particulier des fibres kératiniques humaines telles que les cheveux, en association avec au moins une base d'oxydation.

16. Procédé de teinture d'oxydation des fibres kératiniques et en particulier des fibres kératiniques humaines telles que les cheveux, caractérisé par le fait qu'on applique sur ces fibres au moins une composition tinctoriale telle que définie à l'une quelconque des revendications 1 à 14, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

17. Procédé selon la revendication 16, caractérisé par le fait que l'agent oxydant est choisi parmi le peroxyde d'hydrogène, le peroxyde d'urée, les bromates de métaux alcalins, les persels tels que les perborates et persulfates.

18. Dispositif à plusieurs compartiments, ou "kit" de teinture à plusieurs compartiments, dont un premier compartiment renferme une composition tinctoriale telle que définie à l'une quelconque des revendications 1 à 14 et un second compartiment renferme une composition oxydante.

19. Dérivés N-substitués de la 4-hydroxy indoline de formule (I'), et leurs sels d'addition avec un acide : 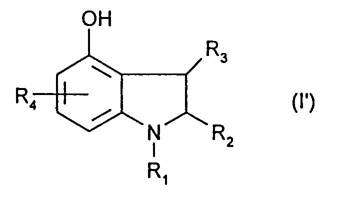 dans laquelle :
- R₁ représente un radical monohydroxyalkyle en C₁-C₄ ; polyhydroxyalkyle en C₂-C₄ ; alcoxy(C₁-C₄)alkyle en C₁-C₄ ; hydroxyalcoxy(C₁-C₄)alkyle en C₁-C₄ ; acétyle, aminoalkyle en C₁-C₄ ; aminoalkyle en C₁-C₄ dont l'amine est mono ou disubstituée par un groupement alkyle en C₁-C₄, par un groupement acétyle, par un groupement monohydroxyalkyle en C₁-C₄ ou par un groupement polyhydroxyalkyle en C₂-C₄ ; alkyle(C₁-C₄)thioalkyle en C₁-C₄, monohydroxyalkyl(C₁-C₄)thioalkyle en C₁-C₄ ; polyhydroxyalkyl(C₂-C₄)thioalkyle en C₁-C₄ ; carboxyalkyle en C₁-C₄ ; alcoxy(C₁-C₄)carbonylalkyle en C₁-C₄, acétylaminoalkyle en C₁-C₄ ; cyanoalkyle en C₁-C₄ ; trifluoroalkyle en C₁-C₄ ; halogénoalkyle en C₁-C₄ ; phosphoalkyle en C₁-C₄ ou sulfoalkyle en C₁-C₄ ;
- R₂ et R₃, identiques ou différents, représentent un atome d'hydrogène ou d'halogène ; un radical alkyle en C₁-C₄ ; carboxyle ; alcoxy(C₁-C₄)carbonyle ou formyle ;
- R₄ représente un atome d'hydrogène ou d'halogène ; un radical alkyle en C₁-C₄ ; alcoxy en C₁-C₄ ; acétylamino ; monohydroxyalkyle en C₁-C₅ ; polyhydroxyalkyle en C₂-C₄ ; alcoxy(C₁-C₄)alkyle en C₁-C₄ ;-thiophène ; furane ; phényle ; aralkyle en C₁-C₄ ; phényle ou aralkyle en C₁-C₄ substitué par un atome d'halogène, un alkyle en C₁-C₄, un trifluorométhyle, un alcoxy en C₁-C₄, un amino ou par un amino mono- ou disubstitué par un radical alkyle en C₁-C₄ ; alkyl(C₁-C₄)aminoalkyle en C₁-C₄ ou dialkyl(C₁-C₄)aminoalkyle en C₁-C₄ ;
étant entendu que :
- lorsque R₂, R₃ et R₄ représentent simultanément un atome d'hydrogène, alors R₁ ne peut représenter un radical méthoxycarbonylpropyle.

20. Dérivés selon la revendication 19, caractérisé par le fait que le substituant R₁ est choisi parmi les radicaux monohydroxyalkyle en C₁-C₄ et les radicaux polyhydroxyalkyle en C₂-C₄.

21. Dérivés selon la revendication 19, caractérisé par le fait qu'ils sont choisis parmi :
- la 4-hydroxy 1-N-(β-hydroxyéthyl) indoline,
- la 4-hydroxy 1-N-(β-hydroxypropyl) indoline,
- la 1-N-acétyl 4-hydroxy indoline,
- la 1-N-(β,γ-dihydroxypropyl) 4-hydroxy indoline,
- la 4-hydroxy 1-N-(β-hydroxyéthyl) 5-méthyl indoline,
- la 4-hydroxy 1-N-(β-hydroxypropyl) 5-méthyl indoline,
- la 1-N-(β,γ-dihydroxypropyl) 4-hydroxy 5-méthyl indoline,
- la 4-hydroxy 1-N-(β-hydroxyéthyl) 6-méthyl indoline,
- la 4-hydroxy 1-N-(β-hydroxypropyl) 6-méthyl indoline,
- la 1-N-(β,γ-dihydroxypropyl) 4-hydroxy 6-méthyl indoline,
- la 5-benzyl 4-hydroxy 1-N-(β-hydroxyéthyl) indoline,
- la 5-benzyl 4-hydroxy 1-N-(β-hydroxypropyl) indoline,
- la 5-benzyl 1-N-(β,γ-dihydroxypropyl) 4-hydroxy indoline,
- la 4-hydroxy 1-N-(β-hydroxyéthyl) 5-β-hydroxyéthyl indoline,
- la 4-hydroxy 5-β-hydroxyéthyl 1-N-(β-hydroxypropyl) indoline,
- la 1-N-(β,γ-dihydroxypropyl) 4-hydroxy 5-β-hydroxyéthyl indoline,
- la 4-hydroxy 1-N-(β-hydroxyéthyl) 5-β,γ-dihydroxypropyl indoline,
- la 4-hydroxy 1-N-(β-hydroxypropyl) 5-β,γ-dihydroxypropyl indoline,
- la 1-N-(β,γ-dihydroxypropyl) 4-hydroxy 5-β,γ-dihydroxypropyl indoline,
- la 1-N-(γ-diméthylaminopropyl) 4-hydroxy indoline,
- la 1-N-éthylaminoéthyl 4-hydroxy indoline
et leurs sels d'addition avec un acide.

22. Dérivés selon la revendication 21, caractérisé par le fait qu'ils sont choisis parmi:
- la 4-hydroxy 1-N-(β-hydroxyéthyl) indoline,
- la 4-hydroxy 1-N-(β-hydroxypropyl) indoline,
- la 1-N-acétyl 4-hydroxy indoline,
- la 1-N-(β,γ-dihydroxypropyl) 4-hydroxy indoline,
- la 4-hydroxy 1-N-(β-hydroxyéthyl) 5-méthyl indoline,
- la 1-N-(γ-diméthylaminopropyl) 4-hydroxy indoline,
et leurs sels d'addition avec un acide.

23. Procédé de préparation des dérivés N-substitués de la 4-hydroxy indoline de formule (I') telle que définie à la revendication 19, caractérisé par le fait qu'il consiste à faire réagir, dans une première étape, un 4-oxo 4,5,6,7-tetrahydro benzofurane avec une amine substituée, dans un milieu solvant dont la température est comprise entre 80 et 160°C, pour conduire à un dérivé 4-oxo 4,5,6,7-tetrahydroindolique, puis dans une deuxième étape, à aromatiser ledit dérivé 4-oxo 4,5,6,7-tetrahydroindolique par déshydrogénation catalytique en milieu solvant, à une température comprise entre 150 et 220°C, pour conduire à un dérivé N-substitué du 4-hydroxy indole de formule (IX) dont la double liaison du noyau pyrrolique est ensuite réduite, dans une troisième étape, pour conduire à un dérivé N-substitué de la 4-hydroxy indoline de formule (I').

## Claims

1. Composition for dyeing keratinous fibres and in particular human keratinous fibres such as hair, characterized in that it comprises, in a medium appropriate for dyeing:
- as coupler, at least one N-substituted 4-hydroxyindoline derivative of formula (I) and/or at least one of its addition salts with an acid: 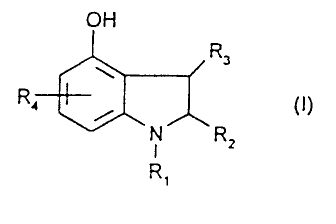 in which:
- R₁ represents a C₁-C₄ monohydroxyalkyl radical; a C₂-C₄ polyhydroxyalkyl radical; a (C₁-C₄)alkoxy(C₁-C₄)alkyl radical; a hydroxy(C₁-C₄)alkoxy(C₁-C₄)alkyl radical; an acetyl or C₁-C₄ aminoalkyl radical; a C₁-C₄ aminoalkyl radical in which the amine is mono- or disubstituted by a C₁-C₄ alkyl group, by an acetyl group, by a C₁-C₄ monohydroxyalkyl group or by a C₂-C₄ polyhydroxyalkyl group; a (C₁-C₄)alkylthio(C₁-C₄)alkyl radical or a monohydroxy(C₁-C₄)alkylthio(C₁-C₄)alkyl radical; a polyhydroxy(C₂-C₄)alkylthio(C₁-C₄)alkyl radical; a C₁-C₄ carboxyalkyl radical; a (C₁-C₄)alkoxycarbonyl(C₁-C₄)alkyl radical or a C₁-C₄ acetylaminoalkyl radical; a C₁-C₄ cyanoalkyl radical; a C₁-C₄ trifluoroalkyl radical; a C₁-C₄ haloalkyl radical; a C₁-C₄ phosphoalkyl radical or a C₁-C₄ sulphoalkyl radical;
- R₂ and R₃, which are identical or different, represent a hydrogen or halogen atom; a C₁-C₄ alkyl radical; a carboxyl radical; a (C₁-C₄)alkoxycarbonyl radical or a formyl radical;
- R₄ represents a hydrogen or halogen atom; a C₁-C₄ alkyl radical; a C₁-C₄ alkoxy radical; an acetylamino radical; a C₁-C₅ monohydroxyalkyl radical; a C₂-C₄ polyhydroxyalkyl radical; a (C₁-C₄)alkoxy(C₁-C₄)alkyl radical; a thiophene radical; a furan radical; a phenyl radical; a C₁-C₄ aralkyl radical; a phenyl or C₁-C₄ aralkyl radical substituted by a halogen atom, a C₁-C₄ alkyl, a trifluoromethyl, a C₁-C₄ alkoxy or an amino or by an amino mono- or disubstituted by a C₁-C₄ alkyl radical; a (C₁-C₄)alkylamino(C₁-C₄)alkyl radical or a di (C₁-C₄) alkylamino (C₁-C₄) alkyl radical; the C₁-C₄ alkyl and C₁-C₄ alkoxy groups being linear or branched and it being possible for the halogen atoms to be chosen from chlorine, bromine, iodine and fluorine,
- and at least one oxidation base.

2. Composition according to Claim 1, characterized in that the substituent R₁ is chosen from the C₁-C₄ monohydroxyalkyl radicals and the C₂-C₄ polyhydroxyalkyl radicals.

3. Composition according to Claim 1, characterized in that the compounds of formula (I) are chosen from:
- 4-hydroxy-1-N-(β-hydroxyethyl)indoline,
- 4-hydroxy-1-N-(β-hydroxypropyl)indoline,
- 1-N-acetyl-4-hydroxyindoline,
- 1-N-(β,γ-dihydroxypropyl)-4-hydroxyindoline,
- 4-hydroxy-1-N-(β-hydroxyethyl)-5-methylindoline,
- 4-hydroxy-1-N-(β-hydroxypropyl)-5-methylindoline,
- 1-N-(β,γ-dihydroxypropyl)-4-hydroxy-5-methylindoline,
- 4-hydroxy-1-N-(β-hydroxyethyl)-6-methylindoline,
- 4-hydroxy-1-N-(β-hydroxypropyl)-6-methylindoline,
- 1-N-(β,γ-dihydroxypropyl)-4-hydroxy-6-methylindoline,
- 5-benzyl-4-hydroxy-1-N-(β-hydroxyethyl)indoline,
- 5-benzyl-4-hydroxy-1-N-(β-hydroxypropyl)indoline,
- 5-benzyl-1-N-(β,γ-dihydroxypropyl)-4-hydroxyindoline,
- 4-hydroxy-1-N-(β-hydroxyethyl)-5-(β-hydroxyethyl)indoline,
- 4-hydroxy-5-(β-hydroxyethyl)-1-N-(β-hydroxypropyl)indoline,
- 1-N-(β,γ-dihydroxypropyl)-4-hydroxy-5-(β-hydroxyethyl)indoline,
- 4-hydroxy-1-N-(β-hydroxyethyl)-5-(β,γ-dihydroxypropyl)indoline,
- 4-hydroxy-1-N-(β-hydroxypropyl)-5-(β,γ-dihydroxypropyl)indoline,
- 1-N-(β,γ-dihydroxypropyl)-4-hydroxy-5-(β,γ-dihydroxypropyl)indoline,
- 1-N-(γ-dimethylaminopropyl)-4-hydroxyindoline,
- 1-N-ethylaminoethyl-4-hydroxyindoline,
and their addition salts with an acid.

4. Composition according to Claim 3, characterized in that the compounds of formula (I) are chosen from:
- 4-hydroxy-1-N-(β-hydroxyethyl)indoline,
- 4-hydroxy-1-N-(β-hydroxypropyl)indoline,
- 1-N-acetyl-4-hydroxyindoline,
- 1-N-(β,γ-dihydroxypropyl)-4-hydroxyindoline,
- 4-hydroxy-1-N-(β-hydroxyethyl)-5-methylindoline,
- 1-N-(γ-dimethylaminopropyl)-4-hydroxyindoline,
and their addition salts with an acid.

5. Composition according to any one of the preceding claims, characterized in that the addition salts with an acid of the compounds of formula (I) are chosen from the hydrochlorides, the hydrobromides, the sulphates and the tartrates.

6. Composition according to any one of the preceding claims, characterized in that the compound or compounds of formula (I) represent from 0.0005 to 12 % by weight of the total weight of the dyeing composition.

7. Composition according to Claim 6, characterized in that the compound or compounds of formula (I) represent from 0.005 to 6 % by weight of the total weight of the dyeing composition.

8. Composition according to any one of the preceding claims, characterized in that the oxidation base or bases are chosen from para-phenylenediamines, bisphenylalkylenediamines, para-aminophenols, orthoaminophenols, heterocyclic bases and their addition salts with an acid.

9. Composition according to any one of the preceding claims, characterized in that the oxidation base or bases represent from 0.0005 to 12 % by weight approximately of the total weight of the dyeing composition.

10. Composition according to Claim 9, characterized in that the oxidation base or bases represent from 0.005 to 6 % by weight approximately of the total weight of the dyeing composition.

11. Composition according to any one of the preceding claims, characterized in that it additionally contains one or more additional couplers other than the N-substituted 4-hydroxyindoline derivatives of formula (I) and/or one or more direct dyes.

12. Composition according to any one of the preceding claims, characterized in that the medium appropriate for the dyeing (or vehicle) is composed of water or of a mixture of water and of at least one organic solvent chosen from lower C₁-C₄ alkanols, glycerol, glycols and glycol ethers, aromatic alcohols, analogous products and their mixtures.

13. Composition according to any one of the preceding claims, characterized in that it exhibits a pH of between 3 and 12.

14. Composition according to any one of the preceding claims, characterized in that it is provided in the form of liquids, creams or gels or in any other form appropriate for carrying out dyeing of keratinous fibres and in particular of human hair.

15. Use of the N-substituted 4-hydroxyindoline derivatives of formula (I) as defined in any one of Claims 1 to 4 as couplers in compositions for the oxidation dyeing of keratinous fibres and in particular of human keratinous fibres, such as hair, in combination with at least one oxidation base.

16. Process for the oxidation dyeing of keratinous fibres and in particular of human keratinous fibres, such as hair, characterized in that at least one dyeing composition as defined in any one of Claims 1 to 14 is applied to these fibres, the colour being developed at acid, neutral or alkaline pH using an oxidizing agent which is added only at the time of use to the dyeing composition or which is present in an oxidizing composition separately applied, simultaneously or sequentially.

17. Process according to Claim 16, characterized in that the oxidizing agent is chosen from hydrogen peroxide, urea hydrogen peroxide, alkali metal bromates, or persalts, such as perborates and persulphates.

18. Multi-compartment device, or multi-compartment dyeing "kit", a first compartment of which contains a dyeing composition as defined in any one of Claims 1 to 14 and a second compartment of which contains an oxidizing composition.

19. N-Substituted 4-hydroxyindoline derivatives of formula (I') and their addition salts with an acid: 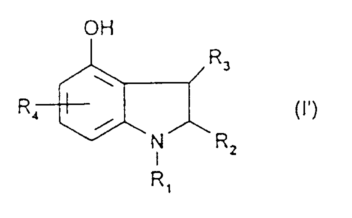 in which:
- R₁ represents a C₁-C₄ monohydroxyalkyl radical; a C₂-C₄ polyhydroxyalkyl radical; a (C₁-C₄)alkoxy(C₁-C₄)alkyl radical; a hydroxy(C₁-C₄)alkoxy(C₁-C₄)alkyl radical; an acetyl or C₁-C₄ aminoalkyl radical; a C₁-C₄ aminoalkyl radical in which the amine is mono- or disubstituted by a C₁-C₄ alkyl group, by an acetyl group, by a C₁-C₄ monohydroxyalkyl group or by a C₂-C₄ polyhydroxyalkyl group; a (C₁-C₄)alkylthio(C₁-C₄)alkyl radical or a monohydroxy(C₁-C₄)alkylthio(C₁-C₄)alkyl radical; a polyhydroxy(C₂-C₄)alkylthio(C₁-C₄)alkyl radical; a C₁-C₄ carboxyalkyl radical; a (C₁-C₄)alkoxycarbonyl(C₁-C₄)alkyl radical or a C₁-C₄ acetylaminoalkyl radical; a C₁-C₄ cyanoalkyl radical; a C₁-C₄ trifluoroalkyl radical; a C₁-C₄ haloalkyl radical; a C₁-C₄ phosphoalkyl radical or a C₁-C₄ sulphoalkyl radical;
- R₂ and R₃, which are identical or different, represent a hydrogen or halogen atom; a C₁-C₄ alkyl radical; a carboxyl radical; a (C₁-C₄)alkoxycarbonyl radical or a formyl radical;
- R₄ represents a hydrogen or halogen atom; a C₁-C₄ alkyl radical; a C₁-C₄ alkoxy radical; an acetylamino radical; a C₁-C₅ monohydroxyalkyl radical; a C₂-C₄ polyhydroxyalkyl radical; a (C₁-C₄)alkoxy(C₁-C₄)alkyl radical; a thiophene radical; a furan radical; a phenyl radical; a C₁-C₄ aralkyl radical; a phenyl or C₁-C₄ aralkyl radical substituted by a halogen atom, a C₁-C₄ alkyl, a trifluoromethyl, a C₁-C₄ alkoxy or an amino or by an amino mono- or disubstituted by a C₁-C₄ alkyl radical; a (C₁-C₄)alkylamino(C₁-C₄)alkyl radical or a di(C₁-C₄)alkylamino(C₁-C₄)alkyl radical;
it being understood that:
- when R₂, R₃ and R₄ simultaneously represent a hydrogen atom, then R₁ cannot represent a methoxycarbonylpropyl radical.

20. Derivatives according to Claim 19, characterized in that the R₁ substituent is chosen from the C₁-C₄ monohydroxyalkyl radicals and the C₂-C₄ polyhydroxyalkyl radicals.

21. Derivatives according to Claim 19, characterized in that they are chosen from:
- 4-hydroxy-1-N-(β-hydroxyethyl)indoline,
- 4-hydroxy-1-N-(β-hydroxypropyl)indoline,
- 1-N-acetyl-4-hydroxyindoline,
- 1-N-(β,γ-dihydroxypropyl)-4-hydroxyindoline,
- 4-hydroxy-1-N-(β-hydroxyethyl)-5-methylindoline,
- 4-hydroxy-1-N-(β-hydroxypropyl)-5-methylindoline,
- 1-N-(β,γ-dihydroxypropyl)-4-hydroxy-5-methylindoline,
- 4-hydroxy-1-N-(β-hydroxyethyl)-6-methylindoline,
- 4-hydroxy-1-N-(β-hydroxypropyl)-6-methylindoline,
- 1-N-(β,γ-dihydroxypropyl)-4-hydroxy-6-methylindoline,
- 5-benzyl-4-hydroxy-1-N-(β-hydroxyethyl)indoline,
- 5-benzyl-4-hydroxy-1-N-(β-hydroxypropyl)indoline,
- 5-benzyl-1-N-(β,γ-dihydroxypropyl)-4-hydroxyindoline,
- 4-hydroxy-1-N-(β-hydroxyethyl)-5-(β-hydroxyethyl)indoline,
- 4-hydroxy-5-(β-hydroxyethyl)-1-N-(β-hydroxypropyl)indoline,
- 1-N-(β,γ-dihydroxypropyl)-4-hydroxy-5-(β-hydroxyethyl)indoline,
- 4-hydroxy-1-N-(β-hydroxyethyl)-5-(β,γ-dihydroxypropyl)indoline,
- 4-hydroxy-1-N-(β-hydroxypropyl)-5-(β,γ-dihydroxypropyl)indoline,
- 1-N-(β,γ-dihydroxypropyl)-4-hydroxy-5-(β,γ-dihydroxypropyl)indoline,
- 1-N-(γ-dimethylaminopropyl)-4-hydroxyindoline,
- 1-N-ethylaminoethyl-4-hydroxyindoline,
and their addition salts with an acid.

22. Derivatives according to Claim 21, characterized in that they are chosen from:
- 4-hydroxy-1-N-(β-hydroxyethyl)indoline,
- 4-hydroxy-1-N-(β-hydroxypropyl)indoline,
- 1-N-acetyl-4-hydroxyindoline,
- 1-N-(β,γ-dihydroxypropyl)-4-hydroxyindoline,
- 4-hydroxy-1-N-(β-hydroxyethyl)-5-methylindoline,
- 1-N-(γ-dimethylaminopropyl)-4-hydroxyindoline,
and their addition salts with an acid.

23. Process for the preparation of the N-substituted 4-hydroxyindoline derivatives of formula (I') as defined in Claim 19, characterized in that it comprises the reaction, in a first stage, of a 4-oxo-4,5,6,7-tetrahydrobenzofuran with a substituted amine, in a solvent medium, the temperature of which is between 80 and 160°C, to result in a 4-oxo-4,5,6,7-tetrahydroindole derivative, and then, in a second stage, the aromatization of the said 4-oxo-4,5,6,7-tetrahydroindole derivative by catalytic dehydrogenation in solvent medium, at a temperature of between 150 and 220°C, to result in an N-substituted 4-hydroxyindole derivative of formula (IX), the double bond of the pyrrole ring of which is then reduced, in a third stage, to result in an N-substituted 4-hydroxyindoline derivative of formula (I').

## Patentansprüche

1. Zusammensetzung zum Färben von Keratinfasern und insbesondere menschlichen Keratinfasern, wie dem Haar,
dadurch gekennzeichnet, daß
sie in einem zum Färben geeigneten Medium enthält:
- als Kuppler mindestens ein N-substituiertes 4-Hydroxyindolinderivat der Formel (I) und/oder mindestens eines der Additionssalze dieser Verbindungen mit einer Säure: 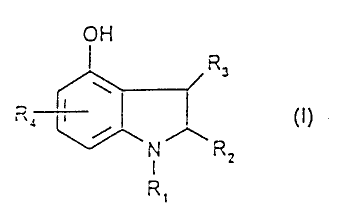 worin bedeuten:
R₁ C₁₋₄-Monohydroxyalkyl, C₂₋₄-Polyhydroxyalkyl, C₁₋₄-Alkoxy-C₁₋₄-alkyl, C₁₋₄-Hydroxyalkoxy- C₁₋₄-alkyl, Acetyl, C₁₋₄-Aminoalkyl, C₁₋₄-Aminoalkyl, wobei die Aminogruppe ein- oder zweifach mit einer C₁₋₄-Alkylgruppe, Acetylgruppe, C₁₋₄-Monohydroxyalkylgruppe oder C₂₋₄-Polyhydroxyalkylgruppe substituiert ist, C₁₋₄-Alkyl-C₁₋₄-thioalkyl, C₁₋₄-Monohydroxyalkyl-C₁₋₄-thioalkyl, C₂₋₄-Polyhydroxyalkyl-C₁₋₄-thioalkyl, C₁₋₄-Carboxyalkyl, C₁₋₄-Alkoxy-C₁₋₄-carbonylalkyl, C₁₋₄-Acetylaminoalkyl, C₁₋₄-Cyanoalkyl, C₁₋₄-Trifluoralkyl, C₁₋₄-Halogenalkyl, C₁₋₄ -Phosphoalkyl oder C₁₋₄-Sulfoalkyl;
R₂ und R₃, die identisch oder voneinander verschieden sind, Wasserstoff, Halogen, C₁₋₄-Alkyl, Carboxy, C₁₋₄-Alkoxycarbonyl oder Formyl;
R₄ Wasserstoff, Halogen, C₁₋₄-Alkyl, C₁₋₄-Alkoxy, Acetylamino, C₁₋₅-Monohydroxyalkyl, C₂₋₄-Polyhydroxyalkyl, C₁₋₄-Alkoxy-C₁₋₄-alkyl, Thiophen, Furan, Phenyl, C₁₋₄-Aralkyl, Phenyl oder C₁₋₄-Aralkyl, die mit Halogen, C₁₋₄-Alkyl, Trifluormethyl, C₁₋₄-Alkoxy, Amino oder Amino, das ein- oder zweifach mit einer C₁₋₄-Alkylgruppe substituiert ist, substituiert sind, C₁₋₄-Alkyl-C₁₋₄-aminoalkyl oder C₁₋₄-Dialkyl-C₁₋₄-aminoalkyl, wobei die C₁₋₄-Alkyl- und C₁₋₄-Alkoxygruppen geradkettig oder verzweigt vorliegen können und die Halogenatome unter Chlor, Brom, Iod und Fluor ausgewählt sein können.
und
- mindestens eine Oxidationsbase.

2. Zusammensetzung nach Anspruch 1, dadurch gekennzeichnet, daβ der Substituent R₁ unter den Gruppen C₁₋₄-Monohydroxyalkyl und C₂₋₄-Polyhydroxyalkyl ausgewählt ist.

3. Zusammensetzung nach Anspruch 1, dadurch gekennzeichnet, daβ die Verbindungen der Formel (I) ausgewählt sind unter:
4-Hydroxy-1-N-(β-hydroxyethyl)-indolin,
4-Hydroxy-1-N-(β-hydroxypropyl)-indolin,
1-N-Acetyl-4-hydroxy-indolin,
1-N-(β,γ-Dihydroxypropyl)-4-hydroxy-indolin,
4-Hydroxy-1-N-(β-hydroxyethyl)-5-methyl-indolin,
4-Hydroxy-1-N-(β-hydroxypropyl)-5-methyl-indolin,
1-N-(β,γ-Dihydroxypropyl)-4-hydroxy-5-methyl-indolin,
4-Hydroxy-1-N-(β-hydroxyethyl)-6-methyl-indolin,
4-Hydroxy-1-N-(β-hydroxypropyl)-6-methyl-indolin,
1-N-(β,γ-Dihydroxypropyl)-4-hydroxy-6-methyl-indolin,
5-Benzyl-4-hydroxy-1-N-(β-hydroxyethyl)-indolin,
5-Benzyl-4-hydroxy-1-N-(β-hydroxypropyl)-indolin,
5-Benzyl-1-N-(β,γ-dihydroxypropyl)-4-hydroxy-indolin,
4-Hydroxy-1-N-(β-hydroxyethyl)-5-ß-hydroxyethyl-indolin,
4-Hydroxy-5-β-hydroxyethyl-1-N-(β-hydroxypropyl)-indolin,
1-N-(β,γ-Dihydroxypropyl)-4-hydroxy-5-β-hydroxyethyl-indolin,
4-Hydroxy-1-N-(β-hydroxyethyl)-5-β,γ-dihydroxypropyl-indolin,
4-Hydroxy-1-N-(β-hydroxvpropyl)-5-β,γ-dihydroxypropyl-indolin,
1-N-(β,γ-Dihydroxypropyl)-4-hydroxy-5-β,γ-dihydroxypropyl-indolin,
1-N-(γ-Dimethylaminopropyl)-4-hydroxy-indolin,
1-N-Ethylaminoethyl-4-hydroxy-indolin, und den Additionssalzen dieser Verbindungen mit einer Säure.

4. Zusammensetzung nach Anspruch 3, dadurch gekennzeichnet, daβ die Verbindungen der Formel (I) ausgewählt sind unter:
4-Hydroxy-1-N-(β-hydroxyethyl)-indolin,
4-Hydroxy-1-N-(β-hydroxypropyl)-indolin,
1-N-Acetyl-4-hydroxy-indolin,
1-N-(β,γ-Dihydroxypropyl)-4-hydroxy-indolin,
4-Hydroxy-1-N-(β-hydroxyethyl)-5-methyl-indolin,
1-N-(γ-Dimethylaminopropyl)-4-hydroxy-indolin und den Additionssalzen dieser Verbindungen mit einer Säure.

5. Zusammensetzung nach einem der vorhergehenden Ansprüche, dadurch gekennzeichnet, daß die Additionssalze der Verbindungen der Formel (I) mit einer Säure unter den Hydrochloriden, Hydrobromiden, Sulfaten und Tartraten ausgewählt sind.

6. Zusammensetzung nach einem der vorhergehenden Ansprüche, dadurch gekennzeichnet, daß die Verbindung oder die Verbindungen der Formel (I) 0,0005 bis 12 Gew.-% des Gesamtgewichts der Färbemittelzusammensetzung ausmachen.

7. Zusammensetzung nach Anspruch 6, dadurch gekennzeichnet, daß die Verbindung oder die Verbindungen der Formel (I) 0,005 bis 6 Gew.-% des Gesamtgewichts der Färbemittelzusammensetzung ausmachen.

8. Zusammensetzung nach einem der vorhergehenden Ansprüche, dadurch gekennzeichnet, daß die Oxidationsbase oder die Oxidationsbasen unter den p-Phenylendiaminen, Bisphenylalkylendiaminen, p-Aminophenolen, o-Aminophenolen, heterocyclischen Basen und den Additionssalzen dieser Verbindungen mit einer Säure ausgewählt sind.

9. Zusammensetzung nach einem der vorhergehenden Ansprüche, dadurch gekennzeichnet, daß die Oxidationsbase oder die Oxidationsbasen etwa 0,0005 bis 12 Gew.-% des Gesamtgewichts der Färbemittelzusammensetzung ausmachen.

10. Zusammensetzung nach Anspruch 9, dadurch gekennzeichnet, daß die Oxidationsbase oder die Oxidationsbasen etwa 0,005 bis 6 Gew.-% des Gesamtgewichts der Färbemittelzusammensetzung ausmachen.

11. Zusammensetzung nach einem der vorhergehenden Ansprüche, dadurch gekennzeichnet, daβ sie ferner einen oder mehrere zusätzliche Kuppler, die von den N-substituierten 4-Hydroxyindolinderivaten der Formel (I) verschieden sind, und/oder einen oder mehrere Direktfarbstoffe enthält.

12. Zusammensetzung nach einem der vorhergehenden Ansprüche, dadurch gekennzeichnet, daß das zum Färben geeignete Medium (oder der Träger) aus Wasser oder einem Gemisch aus Wasser und mindestens einem organischen Lösungsmittel besteht, das unter den niederen C₁₋₄-Alkanolen, Glycerin, den Glykolen und Glykolethern, den aromatischen Alkoholen, analogen Produkten und deren Gemischen ausgewählt ist.

13. Zusammensetzung nach einem der vorhergehenden Ansprüche, dadurch gekennzeichnet, daß sie einen pH-Wert im Bereich von 3 bis 12 aufweist.

14. Zusammensetzung nach einem der vorhergehenden Ansprüche, dadurch gekennzeichnet, daß sie in Form von Flüssigkeiten, Cremes, Gelen oder beliebigen weiteren Formen vorliegt, die dazu geeignet sind, eine Färbung von Keratinfasern und insbesondere menschlichen Keratinfasern durchzuführen.

15. Verwendung von N-substituierten 4-Hydroxyindolinderivaten der Formel (I) nach einem der Ansprüche 1 bis 4 als Kuppler in Kombination mit mindestens einer Oxidationsbase zum Färben von Keratinfasern und insbesondere menschlichen Keratinfasern, wie dem Haar.

16. Verfahren zum oxidativen Färben von Keratinfasern und insbesondere menschlichen Keratinfasern, wie dem Haar, dadurch gekennzeichnet, daß auf die Fasern mindestens eine Färbemittelzusammensetzung nach einem der Ansprüche 1 bis 14 aufgetragen und die Farbe bei einem sauren, neutralen oder alkalischen pH-Wert mit einem Oxidationsmittel entwickelt wird, das bei der Anwendung zu dieser Färbemittelzusammensetzung gegeben wird oder das in einer oxidierenden Zusammensetzung vorliegt, die gleichzeitig oder getrennt davon anschließend aufgetragen wird.

17. Verfahren nach Anspruch 16, dadurch gekennzeichnet, daß das Oxidationsmittel unter Wasserstoffperoxid, Harnstoffperoxid, Alkalimetallbromaten und Salzen von Persäuren, wie Perboraten und Persulfaten, ausgewählt ist.

18. Vorrichtung mit mehreren Abteilungen oder "Kit" zum Färben mit mehreren Abteilungen, wobei eine erste Abteilung eine Färbemittelzusammensetzung nach einem der Ansprüche 1 bis 14 und eine zweite Abteilung eine oxidierende Zusammensetzung enthält.

19. N-substituierte 4-Hydroxyindolinderivate der Formel (I') und die Additionssalze dieser Verbindungen mit einer Säure: worin bedeuten:
R₁ C₁₋₄-Monohydroxyalkyl, C₂₋₄-Polyhydroxyalkyl, C₁₋₄-Alkoxy-C₁₋₄-alkyl, C₁₋₄-Hydroxyalkoxy-C₁₋₄-alkyl, Acetyl, C₁₋₄-Aminoalkyl, C₁₋₄-Aminoalkyl, wobei die Aminogruppe ein- oder zweifach mit einer C₁₋₄-Alkylgruppe, Acetylgruppe, C₁₋₄-Monohydroxyalkylgruppe oder C₂₋₄-Polyhydroxyalkylgruppe substituiert ist, C₁₋₄-Alkyl-C₁₋₄-thioalkyl, C₁₋₄-Monohydroxyalkyl-C₁₋₄-thioalkyl, C₂₋₄-Polyhydroxyalkyl-C₁₋₄-thioalkyl, C₁₋₄-Carboxyalkyl, C₁₋₄-Alkoxy-C₁₋₄-carbonylalkyl, C₁₋₄-Acetylaminoalkyl, C₁₋₄-Cyanoalkyl, C₁₋₄-Trifluoralkyl, C₁₋₄-Halogenalkyl, C₁₋₄-Phosphoalkyl oder C₁₋₄-Sulfoalkyl;
R₂ und R₃, die identisch oder voneinander verschieden sind, Wasserstoff, Halogen, C₁₋₄-Alkyl, Carboxy, C₁₋₄-Alkoxycarbonyl oder Formyl;
R₄ Wasserstoff, Halogen, C₁₋₄-Alkyl, C₁₋₄-Alkoxy, Acetylamino, C₁₋₅-Monohydroxyalkyl, C₂₋₄-Polyhydroxyalkyl, C₁₋₄-Alkoxy-C₁₋₄-alkyl, Thiophen, Furan, Phenyl, C₁₋₄-Aralkyl, Phenyl oder C₁₋₄-Aralkyl, die mit Halogen, C₁₋₄-Alkyl, Trifluormethyl, C₁₋₄-Alkoxy, Amino oder Amino, das ein- oder zweifach mit einer C₁₋₄-Alkylgruppe substituiert ist, substituiert sind, C₁₋₄-Alkyl-C₁₋₄-aminoalkyl oder C₁₋₄-Dialkyl-C₁₋₄-aminoalkyl;
mit der Maßgabe, daß R₁ keine Methoxycarbonylpropylgruppe bedeuten kann, wenn R₂, R₃ und R₄ gleichzeitig Wasserstoff bedeuten.

20. Derivate nach Anspruch 19, dadurch gekennzeichnet, daß der Substituent R₁ unter den Gruppen C₁₋₄-Monohydroxyalkyl und C₂₋₄-Polyhydroxyalkyl ausgewählt ist.

21. Derivate nach Anspruch 19, dadurch gekennzeichnet, daß sie ausgewählt sind unter:
4-Hydroxy-1-N-(β-hydroxyethyl)-indolin,
4-Hydroxy-1-N-(β-hydroxypropyl)-indolin,
1-N-Acetyl-4-hydroxy-indolin,
1-N-(β,γ-Dihydroxyropyl)-4-hydroxy-indolin,
4-Hydroxy-1-N-(β-hydroxyethyl)-5-methyl-indolin,
4-Hydroxy-1-N-(β-hydroxypropyl)-5-methyl-indolin,
1-N-(β,γ-Dihydroxvpropyl)-4-hydroxy-5-methyl-indolin,
4-Hydroxy-1-N-(β-hydroxyethyl)-6-methyl-indolin,
4-Hydroxy-1-N-(β-hydroxvpropyl)-6-methyl-indolin,
1-N-(β,γ-Dihydroxypropyl)-4-hydroxy-6-methyl-indolin,
5-Benzyl-4-hydroxy-1-N-(β-hydroxyethyl)-indolin,
5-Benzyl-4-hydroxy-1-N-(β-hydroxvpropyl)-indolin,
5-Benzyl-1-N-(β,γ-dihydroxypropyl)-4-hydroxy-indolin,
4-Hydroxy-1-N-(β-hydroxyethyl)-5-β-hydroxyethyl-indolin,
4-Hydroxy-5-β-hydroxyethyl-1-N-(β-hydroxypropyl)-indolin,
1-N-(β,γ-Dihydroxypropyl)-4-hydroxy-5-β-hydroxyethyl-indolin,
4-Hydroxy-1-N-(β-hydroxyethyl)-5-β,γ-dihydroxypropyl-indolin,
4-Hydroxy-1-N-(β-hydroxypropyl)-5-β,γ-dihydroxypropyl-indolin,
1-N-(β,γ-Dihydroxvpropyl)-4-hydroxy-5-β,γ-dihydroxypropyl-indolin,
1-N-(γ-Dimethylaminopropyl)-4-hydroxy-indolin,
1-N-Ethylaminoethyl-4-hydroxy-indolin, und
den Additionssalzen dieser Verbindungen mit einer Säure.

22. Derivate nach Anspruch 21, dadurch gekennzeichnet, daß sie ausgewählt sind unter:
4-Hydroxy-1-N-(β-hydroxyethyl)-indolin,
4-Hydroxy-1-N-(β-hydroxypropyl)-indolin,
1-N-Acetyl-4-hydroxy-indolin,
1-N-(β,γ-Dihydroxypropyl)-4-hydroxy-indolin,
4-Hydroxy-1-N-(β-hydroxyethyl)-5-methyl-indolin,
1-N-(γ-Dimethylaminopropyl)-4-hydroxy-indolin und den Additionssalzen dieser Verbindungen mit einer Säure.

23. Verfahren zur Herstellung von N-substituierten 4-Hydroxyindolinderivaten der Formel (I') nach Anspruch 19, dadurch gekennzeichnet, daß in einem ersten Schritt ein 4-Oxo-4,5,6,7-Tetrahydro-benzofuran mit einem substituierten Amin in einem Lösungsmittelmedium einer Temperatur von 80 bis 160 °C umgesetzt wird, wodurch ein 4-Oxo-4,5,6,7-Tetrahydroindolderivat hergestellt wird, worauf in einem zweiten Schritt das 4-Oxo-4,5,6,7-Tetrahydroindol-derivat durch katalytische Dehydrierung in einem Lösungsmittelmedium bei einer Temperatur im Bereich von 150 bis 220 °C aromatisiert wird, wodurch ein N-substituiertes 4-Hydroxyindolderivat der Formel (IX) hergestellt wird, dessen Doppelbindung am Pyrrolring dann in einem dritten Schritt reduziert wird, wodurch ein N-substituiertes 4-Hydroxyindolinderivat der Formel (I') hergestellt wird.
